# EUROPEAN PATENT SPECIFICATION

(11) **EP 4 225 127 B1**
(45) Date of publication and mention of the grant of the patent: **07.01.2026**
(21) Application number: 21798571.2
(22) Date of filing: 07.10.2021
(51) Int. Cl.: A61B 3/00, A61B 3/08, A61B 3/113, G06F 3/01, G06F 3/0346, A61B 5/11, A61B 5/00

(54) **FUNCTIONAL DIFFERENTIAL EXPLORATION AND REHABILITATION OF BINOCULAR MOTOR COORDINATION IN INDIVIDUALS SUFFERING FROM VERTIGO**
FUNKTIONELLE DIFFERENTIELLE EXPLORATION UND REHABILITATION DER BINOKULAREN MOTORISCHEN KOORDINATION IN INDIVIDUEN, DIE AN SCHWINDEL LEIDEN
EXPLORATION ET RÉADAPTATION FONCTIONNELLES DIFFÉRENTIELLES DE LA COORDINATION MOTRICE BINOCULAIRE CHEZ LES PERSONNES SOUFFRANT DE VERTIGES

(30) Priority: 07.10.2020 EP 20306163
(43) Date of publication of application: 16.08.2023
(73) Proprietor: CENTRE NATIONAL DE LA RECHERCHE SCIENTIFIQUE (CNRS), 75016 Paris (FR); Université Paris Cité, 75006 Paris 6 (FR)
(72) Inventor: KAPOULA, Zoi, 75015 Paris (FR)
(74) Representative: Hautier IP
(86) International application number: PCT/EP2021/077721
(87) International publication number: WO 2022/074132

(56) References cited:
- US-A1- 2019 167 095
- CHIN SEONG: "Visual vertigo: Vertigo of oculomotor origin", MEDICAL HYPOTHESES, EDEN PRESS, PENRITH, US, vol. 116, 3 May 2018 (2018-05-03), pages 84 - 95, XP085403357, ISSN: 0306-9877, DOI: 10.1016/J.MEHY.2018.04.025
- BUCCI M P ET AL: "Binocular coordination of saccades in children with vertigo: Dependency on the vergence state", VISION RESEARCH, ELSEVIER, AMSTERDAM, NL, vol. 46, no. 21, 1 October 2006 (2006-10-01), pages 3594 - 3602, XP025010124, ISSN: 0042-6989, [retrieved on 20061001], DOI: 10.1016/J.VISRES.2006.06.001

## Description

### TECHNICAL FIELD OF THE INVENTION

The present invention is in the field of the management of individuals with vertigo, and more particularly to the functional exploration and rehabilitation of ocular motor coordination in such individuals. It relates to a method for measuring binocular motricity parameters and postural parameters in ecologic conditions (both eye viewing targets displaced in the real 3D space) for individuals suffering from vertigo comprising at least two REMOBI tests of eye movements and a posture test, wherein the REMOBI tests are performed using a device physically stimulating vergences or saccades in the real world in three dimensions and contain multiple trials for each type of stimulation, vergences or saccades. The REMOBI eye movement tests are performed once with the individual in seated position and the other time with the individual in standing position. In the latter case, posture is measured simultaneously. It further relates to a method for detecting intrinsic binocular motor abnormalities and/or postural control abnormalities in an individual suffering from vertigo using the same vergence and posture tests. It also relates to a method for improving binocular motor coordination and postural control in an individual suffering from vertigo.

### BACKGROUND ART

According to clinical studies binocular motor coordination problems exist systematically in patients with vertigo. Vergence eye movements are the ones that concern the correct movement of one eye relative to the other so that the angle of the optic axes is adjusted appropriately according to the depth we are fixating (increase the convergence angle for fixating a near target, decrease it for fixating a far target). During saccades (left right eye movements or up down, the eyes should move the same way, and no vergence should be involved. However, vergence errors occur during saccades that are small physiologically in healthy persons, but could be substantial in patients with vertigo. Vergence eye movements and vergence errors during saccades enable to characterize the quality of binocular motor coordination and they are related. Indeed, vergence errors during saccades are large when the vergence eye movements are poor (see Kapoula Z, Morize A, Daniel F, Jonqua F, Orssaud C, Brémond-Gignac D. Objective Evaluation of Vergence Disorders and a Research-Based Novel Method for Vergence Rehabilitation. Transl Vis Sci Technol. 2016 Mar 11;5(2):8.; and Morize A, Brémond-Gignac D, Daniel F, Kapoula Z. Effects of Pure Vergence Training on Initiation and Binocular Coordination of Saccades. Invest Ophthalmol Vis Sci. 2017 Jan 1;58(1):329-342).

In vertigo patients of organic origin vergence is poor (see Kapoula Z et al. PLoS One. 2013 Jun 18;8(6):e66652).

In children with vertigo symptoms vergence is poor even though there is no organic vestibular cause (Bucci MP et al. J Neurol. 2004 Feb;251(2):204-13; Bucci MP et al. Exp Brain Res. 2004 Aug;157(3):286-95; Bucci MP et al. Vision Res. 2006 Oct;46(21):3594-602). Thus, vergence disorder could cause vertigo and reciprocally vestibular pathology could cause vergence disorder and maintain vertigo symptoms.

The document Seong Chin, Visual vertigo: Vertigo of oculomotor origin, Medical Hypotheses, Volume 116, 2018, Pages 84-95, describes how accurate binocular viewing is achieved through the oculomotor system and external ocular muscles and how external ocular muscles affect motor efferent of the body.

In this context, there is a need for new clinical management protocols for individuals suffering from vertigo, that would take into account the symbiotic interaction between vergence and vestibular function.

### SUMMARY OF THE INVENTION

In the context of the present invention, the inventors found that individual suffering from vertigo perform differently in vergence tests performed using REMOBI device (a binocular motor stimulation device configured to specifically and physically stimulate vergences and saccades and comprising visual or audiovisual targets placed in real space at eccentricities and depths of a surface) in single step paradigm, when the vergence tests is performed in seated or standing position (see Example 1). They further found that, after 4 rehabilitation sessions using the REMOBI device in double step paradigm alternating seated and standing position, vergence performances of more than half of the individual with vertigo (8/14) were improved. In particular, decrease in latency, increase in amplitude or velocity, reduction in variability or a better coupling between saccade and vergence was observed, in particular in standing position (see Example 1). Moreover, patients reported a clear benefit in their navigation and space relation. The benefits were particularly prevalent in patients with vestibular asthenopia and Meniere's disease (see Example 1).

In a first aspect, the present invention thus relates to a method for measuring binocular coordination and postural parameters in an individual suffering from vertigo, comprising:
a1) submitting the individual to ocular motor tests comprising two vergence tests, and optionally two saccade tests and/or two combined tests, one being performed with the individual in seated position and the other with the individual in standing position, and recording the eye movements of the left eye and the right eye of the individual during the two vergence tests, and optionally during the two saccade tests and/or two combined tests;
b1) submitting the individual to posture tests comprising:
   i) the vergence test, and optionally the saccade test and/or the combined test, performed in standing position of step a1),
   ii) a first fixation test at near, consisting in fixating a target for 20 to 40 seconds, preferably 30 seconds, at a first distance between 20 and 50 cm,
   iii) a second fixation test at far, consisting in fixating a target for 20 to 40 seconds, preferably 30 seconds, at a second distance between 100 and 200 cm,
   iv) a first vestibulo-ocular reflex (VOR) test at near, consisting in turning the head left and right while keeping fixating a target at a first distance between 20 and 50 cm, and
   v) a second vestibulo-ocular reflex (VOR) test at far, consisting in turning the head left and right while keeping fixating a target at a second distance between 100 and 200 cm,
   and recording the eye movements of the left eye and the right eye of the individual in posture tests i), iv) and v), recording body sway of the individual during the posture tests i) to iii); and recording head rotations during posture tests iv) and v);
c1) calculating, using a digital processing device, the value of one or more binocular coordination parameter from the records obtained in step a1) and the value of a one or more postural parameter from the records obtained in step b1);
wherein the two vergence tests are conducted using a binocular motor stimulation device configured to specifically and physically stimulate vergences and saccades, comprising visual or audiovisual targets placed in real space at eccentricities and depths of a surface; wherein each of the two vergence tests comprises 30 to 50 trials of:
- visually and/or audiovisually stimulating the individual at a point located at the eyes level, in the central axis between the left eye and the right eye, at a first distance, and
- visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved.

In a second aspect, the present invention also relates to a method for detecting intrinsic ocular motor abnormalities and postural control abnormalities in an individual suffering from vertigo, comprising:
a2) providing previously obtained recordings of the eye movements of the left eye and the right eye of the individual during:
   - ocular motor tests comprising two vergence tests, and optionally two saccade tests and/or two combined tests, one being performed with the individual in seated position and the other with the individual in standing position,
   - posture tests comprising:
      iv. a first vestibulo-ocular reflex (VOR) test at near, consisting in turning the head left and right while keeping fixating a target at a first distance between 20 and 50 cm, and
      v. a second vestibulo-ocular reflex (VOR) test at far, consisting in turning the head left and right while keeping fixating a target at a second distance between 100 and 200 cm
b2) providing previously obtained recordings of body sway of the individual during posture tests comprising:
   i) the vergence test, and optionally the saccade test and/or the combined test, performed in standing position of step a1),
   ii) a first fixation test at near, consisting in fixating a target for 20 to 40 seconds, preferably 30 seconds, at a first distance between 20 and 50 cm,
   iii) a second fixation test at far, consisting in fixating a target for 20 to 40 seconds, preferably 30 seconds, at a second distance between 100 and 200 cm,
   iv) a first vestibulo-ocular reflex (VOR) test at near, consisting in turning the head left and right while keeping fixating a target at a first distance between 20 and 50 cm, and
   v) a second vestibulo-ocular reflex (VOR) test at far, consisting in turning the head left and right while keeping fixating a target at a second distance between 100 and 200 cm,
c2) calculating, using a digital processing device, the value(s) of one or more binocular coordination parameter from the records provided in step a2) and the value(s) of one or more postural parameter(s) from the records provided in step b2);
d2) comparing the value(s) calculated in step c2) to one or more reference value(s) obtained in one or more individual(s) not suffering from vertigo;
e2) concluding to the presence of intrinsic ocular motor abnormalities in the individual suffering from vertigo if one or more binocular coordination parameter calculated in step c2) is significantly different from the one or more reference value(s) obtained in one or more individual(s) not suffering from vertigo; and
f2) concluding to the presence of postural control abnormalities in the individual suffering from vertigo if one or more postural parameter(s) calculated in step c2) is significantly different from the one or more reference value(s) obtained in one or more individual(s) not suffering from vertigo;

wherein the two vergence tests from which the recordings are provided in step a2) have been conducted using a binocular motor stimulation device configured to specifically and physically stimulate vergences and saccades, comprising visual or audiovisual targets placed in real space at eccentricities and depths of a surface;
wherein each of the two vergence tests which recordings are provided comprised 30 to 50 trials of:
   - visually and/or audiovisually stimulating the individual at a point located at the eyes level, in the central axis between the left eye and the right eye, at a first distance, and
   - visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved.

In a third aspect, the present invention also relates to a method for improving binocular coordination and postural control in an individual suffering from vertigo, comprising:
a3) submitting the individual to ocular motor tests comprising two vergence tests, and optionally two saccade tests and/or two combined tests, one being performed with the individual in seated position and the other with the individual in standing position, and recording the eye movements of the left eye and the right eye of the individual during the two vergence tests, and optionally during the two saccade tests and/or two combined tests;
b3) submitting the individual to posture tests comprising:
   i) the vergence test, and optionally the saccade test and/or the combined test, performed in standing position of step a1),
   ii) a first fixation test at near, consisting in fixating a target for 20 to 40 seconds, preferably 30 seconds, at a first distance between 20 and 50 cm,
   iii) a second fixation test at far, consisting in fixating a target for 20 to 40 seconds, preferably 30 seconds, at a second distance between 100 and 200 cm,
   iv) a first vestibulo-ocular reflex (VOR) test at near, consisting in turning the head left and right while keeping fixating a target at a first distance between 20 and 50 cm, and
   v) a second vestibulo-ocular reflex (VOR) test at far, consisting in turning the head left and right while keeping fixating a target at a second distance between 100 and 200 cm,
   and recording the eye movements of the left eye and the right eye of the individual in posture tests i), iv) and v), recording body sway of the individual during the posture tests i) to iii); and recording head rotations during posture tests iv) and v);
c3) calculating, using a digital processing device, the value of one or more binocular coordination parameter from the records obtained in step a3) and the value of a one or more postural parameter from the records obtained in step b3);
d3) comparing the value(s) calculated in step c3) to one or more reference value(s) obtained in one or more individual(s) not suffering from vertigo;
e3) concluding to the presence of intrinsic ocular motor abnormalities in the individual suffering from vertigo if one or more binocular coordination parameter calculated in step c3) is significantly different from the one or more reference value(s) obtained in one or more individual(s) not suffering from vertigo; and
f3) concluding to the presence of postural control abnormalities in the individual suffering from vertigo if one or more postural parameter(s) calculated in step c3) is significantly different from the one or more reference value(s) obtained in one or more individual(s) not suffering from vertigo;
g3) submitting the individual to vergence training sessions once or twice a week between 2 to 6 weeks; and
h3) repeating steps a3) to f3), and optionally step g3), until some improvement in the intrinsic ocular motor abnormalities of the individual suffering from vertigo is found;

wherein the vergence tests and the vergence training sessions, and optionally the saccade tests and/or the combined tests, are conducted using a binocular motor stimulation device configured to specifically and physically stimulate vergences and saccades, comprising visual or audiovisual targets placed in real space at eccentricities and depths of a surface;
wherein each of the two vergence tests comprises 30 to 50 trials of:
   - visually and/or audiovisually stimulating the individual at a point located at the eyes level, in the central axis between the left eye and the right eye, at a first distance, and
   - visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved.

### DESCRIPTION OF THE FIGURES

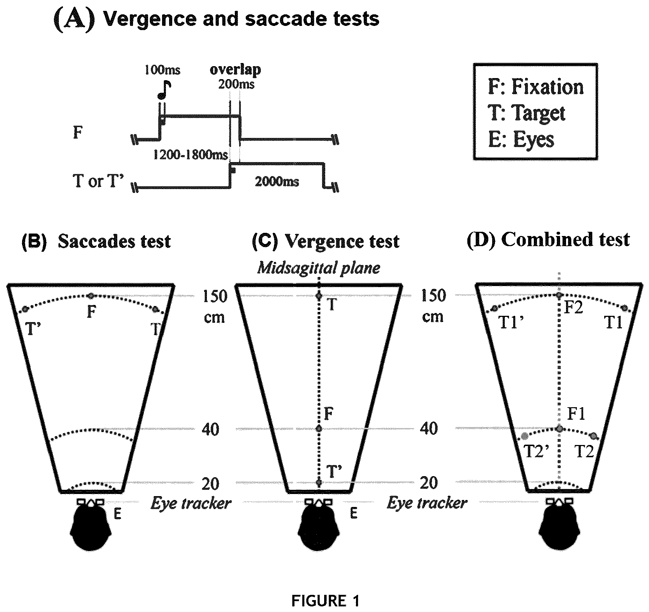

**Figure 1A, 1B, 1C & 1D****:** Vergence and saccade tests experiment Set-Up. Temporal **(A)** and spatial **(B-C)** arrangements of the REMOBI vergence (A, B), saccade (A, C) and combined (A, D) tests. The targets were randomly interleaved in order to test vergence **(B),** saccades **(C)** and combined **(D)** eye movements in different blocks using the temporal paradigm as shown in **(A).** Individuals looked successively at different LEDs; from the initial (40 cm) fixation LED (F), to the target LED for divergence (150 cm, T) or the target LED for convergence (20 cm, T') (B); from the initial (40 cm) fixation LED to the target LED for saccades (20° to left to target T or to the right to target T') (C); and from the initial (40 or 150 cm, F1 or F2) fixation LED to the target LED for combined movement (T1' or T1 when starting from F1, T2' or T2 when starting from F2) (D). Each trial starts with the fixation target that appears for a variable period of 1200 to 1800 ms; following this period the target LED lights are on for 2000ms together with a paired buzzer preceding 50 ms and lasting only 100ms.
**Figure 2**. Method for vergence analysis: convergence and divergence were obtained by subtraction of right eye (RE) position from the left eye (LE) position (LE - RE). The corresponding velocity trace is shown *below.* Mark of onset and offset of vergence is based on velocity criteria: the time point when the eye velocity respectively exceeded or dropped below 5° /s. The *horizontal dotted lines* indicate the targets' (T for divergence, T' for convergence) position.
**Figure 3****.** Method for saccade analysis, illustrated for saccades of 20° in a individual with vergence disorders (TC). All trials are superimposed at t = 0, the instant of target appearance. The bold lines show an individual trial of saccade to the left (A, B): Onset (I) and offset (P) of eye movements were defined according to velocity threshold (V), traces of velocity being illustrated at the bottom (C). Note that coordination of saccades may be evaluated from the disconjugate amplitude (B).
**Figure 4****.** Vergence rehabilitation experiment Set-Up. Temporal (A) and spatial (B, C) arrangements of REMOBI divergence (B) and convergence (C) training blocks. Double-step targets used for divergence and convergence rehabilitation are shown in (B, C) from F to T1 or T2, the first target location; then to T'1 or T'2, the final target location.

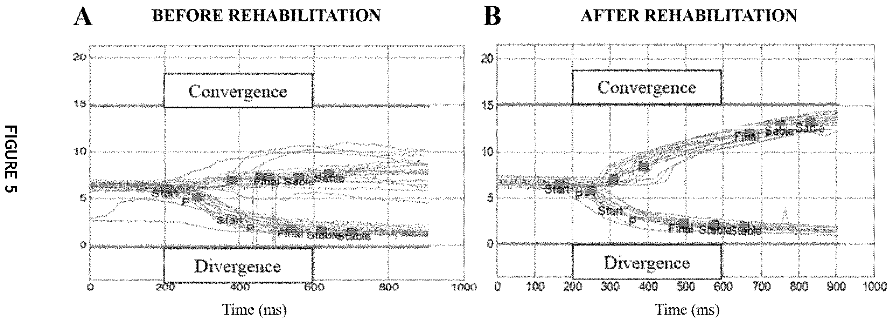

**Figure 5A****:** Trajectories of convergences and divergences from patient 5 (vestibular asthenopia, hypersensitivity) before rehabilitation with REMOBI; the target had appeared at zero time. After a latency of about 200 msec the vergence movement begins. The trajectories are slow, variable and hypsometric especially for the convergence which does not reach its target (indicated by the bold line). **Figure 5B****:** Trajectories of the vergences of the same patient 1 month after the end of the rehabilitation with REMOBI. Convergence trajectories increase to almost reach the target (high bold line), movement dynamics accelerate.
**Figure 6A****:** Trajectories up for convergence targets, down for divergence targets of patient 14 suffering from an old and well documented left Meniere's disease (chronic vertigo, right hyporeflectivity). The trajectory is deficient in both cases (other notations as in Figure 7). **Figure 6B****:** Improved trajectories of the same patient after rehabilitation.
**Figure 7****. (A)** Amplitude of convergence in sitting and standing conditions in vergence test after rehabilitation, **(B)** Amplitude of divergence on sitting and standing conditions vergence test after rehabilitation.
**Figure 8****.** Latency of divergence before and after eye rehabilitation in individuals with vertigo.
**Figure 9A:** Amplitude of divergence in standing vs seated condition. **Figure 9B:** Latency of the divergence in standing position before and after rehabilitation in patients with organic origin of the vertigo (hypo). **Figure 9C:** Amplitude of the divergence in standing position before and after rehabilitation in patients with functional origin of the vertigo (hyper). **Figure 9D:** Latency of the divergence in standing position before and after rehabilitation in patients with functional origin of the vertigo (hyper).
**Figure 10****:** Medio lateral accelerations of vergence standing, eyes open and eyes closed conditions on organic patients.
**Figure 11****:** Correlation between Medio lateral acceleration and amplitude **(A)** and latency **(B)** in hyporeflectivity patients. **(C)** Correlation between Mean power frequency and latency in Hyporeflectivity patients.
**Figure 12****:** Trajectories of convergences and divergences from 2 individuals suffering from chronic vertigo measured using REMOBI device. Expected values and normal average areas of convergences and divergences based on healthy control measurements are shown.
**Figure 13****:** Trajectories of convergences and divergences from 1 individual suffering from Menière's disease measured using REMOBI device. Expected values and normal average areas of convergences and divergences based on healthy control measurements are shown.
**Figure 14****:** Trajectories of convergences and divergences from 2 individuals suffering from vestibular asthenopia measured using REMOBI device. Expected values and normal average areas of convergences and divergences based on healthy control measurements are shown.
**Figure 15****:** Trajectories of convergences and divergences from 1 individual suffering from visual vertigo measured using REMOBI device. Expected values and normal average areas of convergences and divergences based on healthy control measurements are shown.
**Figure 16****:** Trajectories of convergences and divergences from 1 individual suffering from peripheral vertigo measured using REMOBI device. Expected values and normal average areas of convergences and divergences based on healthy control measurements are shown.
**Figure 17****:** Trajectories of convergences and divergences from 1 individual suffering from sensitivity to visual flow measured using REMOBI device. Expected values and normal average areas of convergences and divergences based on healthy control measurements are shown.
**Figure 18****:** Trajectories of convergences and divergences from 2 individuals suffering from positional vertigo measured using REMOBI device. Expected values and normal average areas of convergences and divergences based on healthy control measurements are shown.
**Figure 19****:** Trajectories of convergences and divergences from a healthy individual measured using REMOBI device. Expected values and normal average areas of convergences and divergences based on healthy control measurements are shown.

### DETAILED DESCRIPTION OF THE INVENTION

### Method for measuring binocular motricity and postural parameters in an individual suffering from vertigo

The present invention relates to a first method for measuring binocular coordination and postural parameters in an individual suffering from vertigo, comprising:
a1) submitting the individual to ocular motor tests comprising two vergence tests, and optionally two saccade tests and/or two combined tests, one being performed with the individual in seated position and the other with the individual in standing position, and recording the eye movements of the left eye and the right eye of the individual during the two vergence tests, and optionally during the two saccade tests and/or two combined tests,
b1) submitting the individual to posture tests comprising:
   i) the vergence test, and optionally the saccade test and/or the combined test, performed in standing position of step a1),
   ii) a first fixation test at near, consisting in fixating a target for 20 to 40 seconds, preferably 30 seconds, at a first distance between 20 and 50 cm,
   iii) a second fixation test at far, consisting in fixating a target for 20 to 40 seconds, preferably 30 seconds, at a second distance between 100 and 200 cm,
   iv) a first vestibulo-ocular reflex (VOR) test at near, consisting in turning the head left and right while keeping fixating a target at a first distance between 20 and 50 cm, and
   v) a second vestibulo-ocular reflex (VOR) test at far, consisting in turning the head left and right while keeping fixating a target at a second distance between 100 and 200 cm,
   and recording the eye movements of the left eye and the right eye of the individual in posture tests i), iv) and v), recording body sway of the individual during the posture tests i) to iii); and recording head rotations during posture tests iv) and v);
c1) calculating, using a digital processing device, the value of one or more binocular coordination parameter from the records obtained in step a1) and the value of a one or more postural parameter from the records obtained in step b1);

wherein the two vergence tests are conducted using a binocular motor stimulation device configured to specifically and physically stimulate vergences and saccades, comprising visual or audiovisual targets placed in real space at eccentricities and depths of a surface;
wherein each of the two vergence tests comprises 30 to 50 trials of:
   - visually and/or audiovisually stimulating the individual at a point located at the eyes level, in the central axis between the left eye and the right eye, at a first distance, and
   - visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved.

The tests performed in steps a1) and b1) may be performed in any order and may be randomly interleaved.

### Step a1): ocular motor tests

Step a1) comprises submitting the individual to ocular motor tests comprising two vergence tests and optionally two saccade tests and/or two combined tests, one being performed with the individual in seated position and the other with the individual in standing position, and recording the eye movements of the left eye and the right eye of the individual during the vergence tests, and optionally during the two saccade tests and/or two combined tests.

In addition to the two systematic vergence tests, further ocular motor tests may thus be performed and the eyes movement of the individual recorded during the tests in step a1), including two saccade tests, two combined tests or their combination, all of which are performed once with the individual in seated position and once with the individual in standing position.

The standing position is preferably an orthostatic quiet stance position.

### Vergence tests

The vergence test comprises 30 to 50 trials of:
- visually and/or audiovisually stimulating the individual at a point located at the eyes level, in the central axis between the left eye and the right eye, at a first distance (illustrated for instance by point **F** in **Figure 1C****),** and
- visually and/or audiovisually stimulating the individual at another point located at the eyes's level, in the central axis between the left eye and the right eye, at another distance calling for a convergence movement (illustrated for instance by point T' in **Figure 1C****)** or a divergence movement (illustrated for instance by point T in **Figure 1C****),** half of the trials calling for a convergence movement, the other half for a divergence movement, the trials calling for convergence and divergence movements being interleaved.

The vergence test comprises 30 to 50 trials, preferably an even number of trials between 30 and 50, more preferably between 36 and 44 trials, such as 36, 38, 40, 42 or 44 trials, in particular 40 trials.

Each trial of a vergence test preferably comprises:
- visually and/or audiovisually stimulating the individual at a point located at the eyes level, in the central axis between the left eye and the right eye, at a distance of 40 cm ± 5 cm (i.e. between 35 and 45 cm, preferably between 36 and 44 cm, between 37 and 43 cm, between 38 and 42 cm, between 39 and 41 cm, more preferably 40 cm, see point F in **Figure 1C****)** for a period varying from 1400ms to 2000ms, and
- visually and/or audiovisually stimulating the individual at another point located at the eyes level, in the central axis between the left eye and the right eye, at a distance of 20 cm ± 5 cm (i.e. between 15 and 25 cm, preferably between 16 and 24 cm, between 17 and 23 cm, between 18 and 22 cm, between 19 and 21 cm, more preferably 20 cm, see point T' in Figure 1C) calling for a convergence movement or at a distance of 70 to 150 cm (in particular 70 cm ± 5 cm, i.e. between 65 and 75 cm, preferably between 66 and 74 cm, between 67 and 73 cm, between 68 and 72 cm, between 69 and 71 cm, more preferably 70 cm; or 100 cm ± 5 cm, i.e. between 95 and 105 cm, preferably between 96 and 104 cm, between 97 and 103 cm, between 98 and 102 cm, between 99 and 101 cm, more preferably 100 cm; or 150 cm ± 5 cm, i.e. between 145 and 155 cm, preferably between 146 and 154 cm, between 147 and 153 cm, between 148 and 152 cm, between 14 and 151 cm, more preferably 150 cm; see point T in Figure 1C) calling for a divergence movement during 1500 to 2500 ms, preferably 2000 ms, half of the trials calling for a convergence movement, the other half for a divergence movement, the trials calling for convergence and divergence movements being interleaved.

### Optional saccade tests

In a preferred embodiment, step a1) of the method for measuring binocular motricity and body movement parameters in an individual suffering from vertigo further comprises submitting the individual to two saccade tests, one being performed with the individual in seated position and the other with the individual in standing position, and recording the eye movements of the left eye and the right eye and body sway of the individual during the two saccade tests, wherein the two saccade tests are conducted using a binocular motor stimulation device configured to specifically and physically stimulate vergences and saccades, comprising visual or audiovisual targets placed in real space at eccentricities and depths of a surface, wherein each of the two saccade tests comprises 30 to 50 trials of:
- visually and/or audiovisually stimulating the individual at a point located at the eyes level, in the central axis between the left eye and the right eye (illustrated for instance by point F in **Figure 1B****),** and
- visually and/or audiovisually stimulating the individual at another point located at the eyes level, on the left (illustrated for instance by point T' in **Figure 1B****)** or on the right (illustrated for instance by point T in **Figure 1B****)** of the previous point, half of the trials being on the left, the other half on the right, the trials on the left and on the right being interleaved.

The saccade test comprises 30 to 50 trials, preferably an even number of trials between 30 and 50, more preferably between 36 and 44 trials, such as 36, 38, 40, 42 or 44 trials, in particular 40 trials.

Each trial of a saccade test more preferably comprises:
- visually and/or audiovisually stimulating the individual at a point located at the eyes level, in the central axis between the left eye and the right eye, at a distance of 20, 40, 70 or 100 cm (see point F in **Figure 1B****)** corresponding to a vergence angle of 17°, 9°, 5.3° or 3.72° for a period varying from 1400ms to 2000ms, and
- visually and/or audiovisually stimulating the individual at another point located at the eyes level at the same isovergence arc, at 15° to 20°, preferably 20° of eccentricity on the left (see point T' in Figure 1B) or on the right (see point T in **Figure 1B****)** during 1500 ms to 2000ms, preferably 2000 ms, half of the trials being on the left, the other half on the right, the trials on the left and on the right being interleaved.

### Optional combined tests

In a preferred embodiment, step a1) of the method for measuring binocular motricity and body movement parameters in an individual suffering from vertigo further comprises submitting the individual to two combined tests combining vergence and saccade movements, one being performed with the individual in seated position and the other with the individual in standing position, and recording the eye movements of the left eye and the right eye and body sway of the individual during the two combined tests, wherein the two combined tests are conducted using a binocular motor stimulation device configured to specifically and physically stimulate vergences and saccades, comprising visual or audiovisual targets placed in real space at eccentricities and depths of a surface,
wherein each of the two combined tests comprises 70 to 90 trials of:
   - visually and/or audiovisually stimulating the individual at a point located at the eyes level, in the central axis between the left eye and the right eye, at a distance of 40 cm ± 5 cm (i.e. between 35 and 45 cm, preferably between 36 and 44 cm, between 37 and 43 cm, between 38 and 42 cm, between 39 and 41 cm, more preferably 40 cm, see point F1 in **Figure 1D****)** or at a distance of 150 cm ± 5 cm (i.e. between 35 and 45 cm, preferably between 36 and 44 cm, between 37 and 43 cm, between 38 and 42 cm, between 39 and 41 cm, more preferably 40 cm, see point F2 in **Figure 1D****)** for a period varying from 1400ms to 2000ms, and
   - visually and/or audiovisually stimulating the individual during 1500 ms to 2000ms, preferably 2000 ms at another point located at the eyes level, either
      o when starting from fixation point F1: at a distance of 150 cm ± 5 cm (i.e. between 35 and 45 cm, preferably between 36 and 44 cm, between 37 and 43 cm, between 38 and 42 cm, between 39 and 41 cm, more preferably 40 cm) and at 15° to 20°, preferably 20° of eccentricity on the left (see point T1' in **Figure 1D****)** or on the right (see point T1 in **Figure 1D****),**
      o when starting from fixation point F2: at a distance of 40 cm ± 5 cm (i.e. between 35 and 45 cm, preferably between 36 and 44 cm, between 37 and 43 cm, between 38 and 42 cm, between 39 and 41 cm, more preferably 40 cm) and at 15° to 20°, preferably 20° of eccentricity on the left (see point T2' in **Figure 1D****)** or on the right (see point T2 in **Figure 1D****)**
wherein a quarter of the trials combine convergence and left saccade, a quarter of the trials combine convergence and right saccade, a quarter of the trials combine divergence and left saccade, and a quarter of the trials combine divergence and right saccade, the trials being (preferably randomly) interleaved.

### Optional saccade and combined tests

In a preferred embodiment, the individual is submitted in step a1) to two vergence tests, two saccade tests, and two combined tests (all as described above), one of each type of test being performed with the individual in seated position and the other with the individual in standing position, all tests being conducted using a binocular motor stimulation device configured to specifically and physically stimulate vergences and saccades, comprising visual or audiovisual targets placed in real space at eccentricities and depths of a surface.

### Device for stimulating vergence, saccade, and combined movements

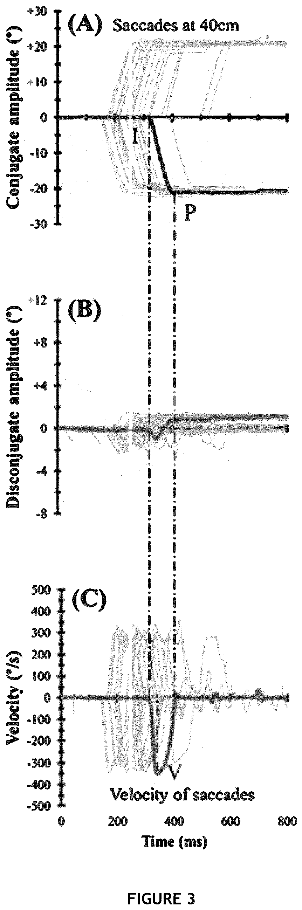

Any binocular motor stimulation device configured to specifically and physically stimulate vergences and saccades and comprising visual or audiovisual targets placed in real space at eccentricities and depths of a surface may be used for the vergence test(s). A device for stimulating binocular motricity by sensory stimulation means such as diodes (visual stimulation) and optionally sound diffusion means (audio stimulation), which are arranged along different arcs of iso-vergence (an arc of iso-vergence is such that the vergence angle of both eyes required to fix each point on this arch is the same) in front of a person may notably be used. This device advantageously comprises four arcs of iso-vergence. Preferably, the sensory stimulation means are arranged on a flat support. The means for sensory stimulation are arranged on arcs at a distance of between 20 and 150 cm from an individual and between each means of stimulation placed on the same arc there is an angle of 10 degrees. The support is advantageously mounted on a height-adjustable stand allowing the device to be properly positioned at the level of the face of an individual. The support for the sensory stimulation means is preferably trapezoidal and has suitable dimensions to test the entire natural range of movements of the eyes (saccades at different depths of iso-vergence arcs, vergences along the median plane, movements combining saccades and vergences) and rehabilitate binocular motricity in direction (saccades), in depth (vergences) or combining them. The stimulation offered by this type of device is flexible, it can be audiovisual (thanks to light diodes and sound means), only audio or only visual. The means for visual and audio stimulation constitute targets for the person. Each sensory stimulation medium is represented by a A preferred such device is the REMOBI^{®} device, which structure is notably described in US8851669 and WO2011073288A1, the content of which are herein incorporated by reference. In particular, **Figure 3** of US8851669 and WO2011073288A1 illustrate a bottom view of a device for the rehabilitation and/or training device for the binocular motivity of a patient according to the invention (display means for visual stimuli and means of generating auditory stimuli).

Due to the presence in the REMOBI^{®} device at quasi the same locations of both visual and audio targets (it is then referred to audiovisual targets), it is possible to perform vergence and saccade tests either using audiovisual stimulation (in this case, each stimulation involves both a visual signal and an audio signal) or using visual stimulation only (in this case, each stimulation involves only a visual signal, but no audio signal).

In the case of audiovisual stimulation, the visual signal and the audio signal start preferably simultaneously or very near, typically the audio signal precedes the visual signal by 50 msec and its duration is shorter (about 50-200 ms, preferably about 100 ms) than the visual signal (generally more than 1000 ms).

### Recording of eye movements

In step a1), the eye movements of the left eye and the right eye of the individual during the ocular motor tests are recorded.

This may be performed using any appropriate eye tracker (also referred to as an eye movement acquisition and recording system). The eye movement acquisition and recording system works advantageously with a video-oculography system, i.e. micro-cameras that focus their lenses on both eyes and record their movements when the person is looking at the device to stimulate binocular motricity (vergence and saccade tests) or when he or she is reading (reading test).

Such a system is for example an eyetracker from the company Pupil Labs^{™}, such as the head mounted device Pupil Core, enabling binocular recording at 200 Hz per eye (https: / /pupil-labs.com/products/core/)

Another type of eye movement acquisition system is the Powref III from the company PlusOptix^{™}. Such a type of system is placed at a distance from the person, for example in front of the person on the REMOBI support. This system allows to measure the eye movement but also, at a distance, the change of eye accommodation for and to plot eye positions but also the plot of the change of eye accommodation. In this regard, one can refer to the document (https://www.medicus.ua/eng/product/ophthalmic-equipment/plusoptiX-R09-PowerRef-3/manufacturer:, 8 "HSOA Journal of Clinical Studies and Medical Case Reports", Kapoula Z, et al, J Clin Stud Med Case Rep 2019, 6: 74). Other examples of suitable eye trackers include the EyeSeeCam system (University of Munich Hospital, Clinical Neuroscience, Munich, Germany, available in the public domain at http://eyeseecam.com/,) or other remote devices e.g. Tobbii (https://www.tobii.com/), Powref (https://plusoptix.com/home).

### Step b1): posture tests

Step b1) comprises submitting the individual to posture tests and recording the body sway and in some posture tests also recording eye movements of the left eye and the right eye of the individual during the posture tests.

The posture tests comprise:
i) the vergence test, and optionally the saccade test and/or the combined test, performed in standing position of step a1),
ii) a first fixation test at near, consisting in fixating a target for 20 to 40 seconds, preferably 30 seconds, at a first distance between 20 and 50 cm,
iii) a second fixation test at far, consisting in fixating a target for 20 to 40 seconds, preferably 30 seconds, at a second distance between 100 and 200 cm,
iv) a first vestibulo-ocular reflex (VOR) test at near, consisting in turning the head left and right while keeping fixating a target at a first distance between 20 and 50 cm, and
v) a second vestibulo-ocular reflex (VOR) test at far, consisting in turning the head left and right while keeping fixating a target at a second distance between 100 and 200 cm.

### Posture test i)

Posture test i) is made simultaneously with the vergence test, and optionally the saccade test and/or the combined test, performed in standing position of step a1), during which not only eye movements of the left eye and the right eye of the individual but also body sway are recorded.

### Posture test ii): first fixation test at near

In posture test ii), the individual is requested to fix a target during 20 to 40 seconds, preferably 30 seconds, at near, i.e. at a first distance between 20 and 50 cm, preferably between 20 and 40 cm, preferably 20, 30, or 40 cm. During a usual fixation test at near, the individual is requested to fix a target during 30 seconds at a first distance of 20, 30 or 40 cm.

During fixation, body sway is recorded using a device comprising three orthogonally mounted accelerometers in transverse, sagittal and coronal planes.

### Posture test iii): second fixation test at far

In posture test iii), the individual is requested to fix a target during 20 to 40 seconds, preferably 30 seconds, at far, i.e. at a first distance between 100 and 200 cm, preferably between 100 and 150 cm, such as 100, 110, 120, 130, 140 or 150 cm, preferably 100 or 150 cm. During a usual fixation test at far, the individual is requested to fix a target during 30 seconds at a second distance of 100 or 150 cm.

During fixation, body sway is recorded using a device comprising three orthogonally mounted accelerometers in transverse, sagittal and coronal planes.

### Posture test iv): first vestibulo-ocular reflex (VOR) test at near

The vestibulo-ocular reflex (VOR) is a reflex acting to stabilize gaze during head movement, with eye movement due to activation of the vestibular system. The reflex acts to stabilize images on the retinas of the eye during head movement, holding gaze is held steadily on a location, by producing eye movements in the direction opposite to head movement.^{[} For example, when the head moves to the right, the eyes move to the left, meaning the image a person sees stays the same even though the head has turned. Since slight head movement is present all the time, VOR is necessary for stabilizing vision: people with an impaired reflex find it difficult to read using print, because the eyes do not stabilise during small head tremors, and also because damage to reflex can cause nystagmus.

In posture test iv), the individual is requested to turn his/her head alternatively left and right while keeping fixating a target at a first distance between 20 and 50 cm, preferably between 20 and 40 cm, preferably 20, 30, or 40 cm. At least 10 movements to the left and right are performed.

During the test, both eye movements of the left eye and the right eye and head rotation movements are recorded. Eye movements of the left eye and the right eye are recorded as disclosed in step a1), while head rotations are recorded using a head mounted accelerometer.

### Posture test v): second vestibulo-ocular reflex (VOR) test at far

In posture test v), the individual is requested to turn his/her head alternatively left and right while keeping fixating a target at a first distance between 100 and 200 cm, preferably between 100 and 150 cm, such as 100, 110, 120, 130, 140 or 150 cm, preferably 100 or 150 cm. At least 10 movements to the left and right are performed.

During the test, both eye movements of the left eye and the right eye and head rotation movements are recorded. Eye movements of the left eye and the right eye are recorded as disclosed in step a1), while head rotations are recorded using a head mounted accelerometer.

### Recording of body sway

The recording of body sway is made using a device comprising three orthogonally mounted accelerometers in transverse, sagittal and coronal planes.

An example of such device is the small DynaPort MiniMod ^{®} (McRoberts B.V. The Hague, The Netherlands) device (74 g) equipped with three intransverse, sagittal and coronal orthogonally mounted accelerometers (AXXL202, AnalogueDevices, Norwood MA, USA). The device is preferably placed on a belt at the lumbosacral level and near the center of mass of the body.

### Recording of head rotations

The recording of head rotations is made using a head mounted accelerometer. In this case, an accelerometer is mounted on the individual's head and acceleration of the individual's head is recorded simultaneously with the individual's eyes movements. Measure and recording of the individual's head acceleration permits to analyze the individual's head stability during the eye movement tests.

Appropriate accelerometers include, without limitation, i.e. XMPU6050 (accelerometer + gyroscope) Bluetooth module.

### Step c1): calculation of one or more binocular coordination parameter and one or more body movement parameter

In step c1), one or more binocular coordination parameter and one or more body movement parameter are calculated using a digital processing device.

### Binocular coordination parameters

### Calculating vergence parameters during vergence and combined tests

A vergence is the simultaneous movement of both eyes in opposite directions to obtain or maintain single binocular vision. To look at an object closer by, the eyes rotate towards each other (convergence), while for an object farther away they rotate away from each other (divergence).

Based on the recording of eye movements made by the eye tracker, the effective trajectory corresponding to each vergence is obtained by calculating an unconjugated signal defined by the difference of the position of the left eye with the position of the right eye (i.e., left eye - right eye).

Each vergence effective trajectory is calculated between the time point when a visual or audiovisual stimulation inducing vergence movement starts and the time point when a next visual or audiovisual stimulation inducing vergence movement starts.

In any vergence test, a representative vergence trajectory corresponding to a mean trajectory of all effective trajectories is calculated, and effective trajectory at too much variance with the representative trajectory may be discarded for further analysis.

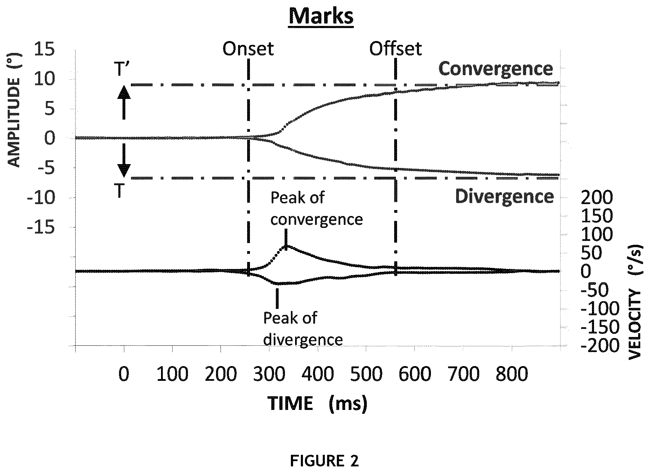

For each vergence effective trajectory, a corresponding effective velocity trajectory is further calculated by conventional calculation (see Figure 2).

Each effective velocity trajectory first permits to calculate the peak velocity of vergence (divergence or convergence), which is defined as the highest velocity value of the effective velocity trajectory (see Figure 2).

For each vergence effective trajectory and corresponding velocity trajectory, several parts of the vergence effective trajectory and specific time points may then be calculated (see Figure 2):
- Onset of vergence (convergence or divergence):
   This parameter is defined as the time point when the vergence velocity, initially close to zero has increased to reach 8-12%, preferably 10%, of the peak velocity (highest velocity in the effective trajectory), or when the vergence velocity initially close to zero has increased to reach a predefined velocity. For instance, when using a vergence test involving a convergence or divergence of about 7-9°, onset of vergence may be defined as the time point when the vergence velocity reaches 4-6° /s, preferably 5° /s.
- Offset of vergence (convergence or divergence):
   This parameter is defined as the time point when the vergence velocity, after decreasing from the peak velocity reaches only 8-12%, preferably 10%, of the peak velocity, or when the vergence velocity after decreasing from the peak velocity reaches a predefined velocity. For instance, when using a vergence test involving a convergence or divergence of about 7-9°, offset of vergence may be defined as the time point when the vergence velocity once more reaches 4-6° /s, preferably 5° /s after decreasing from peak velocity.
- Latency trajectory part (convergence or divergence):
   This part of the effective vergence trajectory corresponds to the part between the time point when the visual or audiovisual stimulation starts (beginning of the trajectory) and convergence or divergence onset.
- Initial phasic trajectory part of vergence or "initial phasic component":
   This part of the effective vergence trajectory corresponds to the part between convergence or divergence onset and offset.
- 80 ms slower trajectory part of vergence or "80 ms component":
   This part of the effective vergence trajectory corresponds to the 80 ms after convergence or divergence offset.
- 160 ms slower trajectory part of vergence or "160 ms component":
   This part of the effective vergence trajectory corresponds to the 160 ms after convergence or divergence offset.

Based on the above-defined time points and trajectory parts, vergence parameters may be calculated. Vergence parameters include:
- Duration parameters:
   o Latency duration of vergence (convergence or divergence):
      This parameter is defined as the duration between the time point when the visual or audiovisual stimulation starts and convergence or divergence onset.
   ∘ Initial phasic duration of vergence (convergence or divergence):
      This parameter is defined as the duration of the initial phasic trajectory part of vergence, i.e. the duration between convergence or divergence onset and offset.
   o Total duration:
      This parameter is defined as the sum of the initial phasic duration of vergence and the following 160 ms.
- Amplitude parameters:
   o Amplitude of the initial phasic component:
      This parameter is defined as (unconjugated signal at offset - unconjugated at signal onset).
   ∘ Amplitude of the 80 ms component:
      This parameter is defined as (unconjugated signal 80 ms after offset-unconjugated signal at offset).
   ∘ Amplitude of the 160 ms component:
      This parameter is defined as (unconjugated signal 160 ms after offset-unconjugated signal at offset).
   ∘ Total amplitude:
      This parameter is defined as the sum of the amplitude of the initial phasic component and the amplitude of the 160 ms component.
- Velocity parameters:
   o Peak velocity:
      As mentioned above, peak velocity is the highest velocity value of the effective velocity trajectory.
      Peak velocity of specific parts of each vergence effective trajectory may also be calculated, including the peak velocity of the initial phasic component. However, it should be noted that peak velocity is clearly found during the initial phasic component and is thus equal to the peak velocity of the initial phasic component.
   ∘ Average velocity of the initial phasic component:
      This parameter is defined as the ratio between the amplitude and the duration of the initial phasic component.
   ∘ Total average velocity:
      This parameter is defined as the ratio between the total amplitude and the total duration, as defined above.

Vergence parameters are preferably calculated separately for divergence and convergence trajectories.

No matter which vergence parameter(s) is(are) calculated, the calculation may be made using any suitable software able to compute vergence effective trajectories from the recordings made by the eye tracker and calculate vergence parameters. Such software may preferably be the AIDEAL^{®} software disclosed in French patent application filed on May 14, 2020 under number FR2004768, the content of which is herein incorporated by reference.

### Calculating saccade parameters during saccade and combined tests

A saccade is a quick, simultaneous movement of both eyes between two or more phases of fixation in the same direction.

Based on the recording of eye movements made by the eye tracker, the effective trajectory corresponding to a saccade is obtained by calculating a conjugate signal defined by the average of the position of the left eye with the position of the right eye (i.e. left+right eyes positions/2).

Each saccade effective trajectory is calculated between the time point when a visual or audiovisual stimulation inducing saccade movement starts and the time point when a next visual or audiovisual stimulation inducing saccade movement starts.

In any saccade test, a representative saccade trajectory corresponding to a mean trajectory of all effective trajectories is calculated, and effective trajectory at too much variance with the representative trajectory may be discarded for further analysis.

For each saccade effective trajectory, a corresponding effective velocity trajectory is further calculated by conventional calculation (see Figure 3, illustrating this for a individual with a vergence disorder tested for saccades of 40° ).

Each effective velocity trajectory first permits to calculate the peak velocity of saccade (right or left), which is defined as the highest velocity value of the effective velocity trajectory (see Figure 3).

For each saccade effective trajectory and corresponding velocity trajectory, several parts of the saccade effective trajectory and specific time points may then be calculated (see Figure 3):
- Onset of saccade (right or left):
   This parameter is defined as the time point when the saccade velocity, initially close to zero has increased to reach 8-12%, preferably 10%, of the peak velocity (highest velocity in the effective trajectory), or when the saccade velocity initially close to zero has increased to reach a predefined velocity. For instance, when using a saccade test involving a right or left saccade of about 20°, onset of saccade may be defined as the time point when the saccade velocity reaches 40-50° /s, preferably 45° /s.
- Offset of saccade (right or left):
   This parameter is defined as the time point when the saccade velocity, after decreasing from the peak velocity reaches only 8-12%, preferably 10%, of the peak velocity, or when the saccade velocity after decreasing from the peak velocity reaches a predefined velocity. For instance, when using a saccade test involving a right or left saccade of about 20°, offset of saccade may be defined as the time point when the saccade velocity once more reaches 40-50° /s , preferably 45° /s after decreasing from peak velocity.
- Latency trajectory part (right or left):
   This part of the effective saccade trajectory corresponds to the part between the time point when the visual or audiovisual stimulation starts (beginning of the trajectory) and right or left saccade onset.
- Initial phasic trajectory part of saccade or "initial phasic component":
   This part of the effective saccade trajectory corresponds to the part between right or left saccade onset and offset.
- 80 ms drift trajectory part of saccade or "80 ms component":
   This part of the effective saccade trajectory corresponds to the 80 ms after right or left saccade offset.
- 160 ms drift trajectory part of saccade or "160 ms component":
   This part of the effective saccade trajectory corresponds to the 160 ms after right or left saccade offset.

Based on the above-defined time points and trajectory parts, saccade parameters may be calculated. Saccade parameters include:
- Duration parameters:
   o Latency duration of saccade (right or left):
      This parameter is defined as the duration between the time point when the visual or audiovisual stimulation starts and right or left saccade onset.
   ∘ Initial phasic duration of saccade (right or left):
      This parameter is defined as the duration of the initial phasic trajectory part of saccade, i.e. the duration between right or left saccade onset and offset.
   o Total duration:
      This parameter is defined as the sum of the initial phasic duration of saccade and the following 160 ms.
- Amplitude parameters:
   o Amplitude of the initial phasic component:
      This parameter is defined as (conjugated signal at offset - conjugated at signal onset)..
   o Amplitude of the 80 ms component:
      This parameter is defined as (conjugated signal 80 ms after offset - conjugated signal at offset).
   ∘ Amplitude of the 160 ms component:
      This parameter is defined as (conjugated signal 160 ms after offset - conjugated signal at offset).
   ∘ Total amplitude:
      This parameter is defined as the sum of the amplitude of the initial phasic component and the amplitude of the 160 ms component.
- Velocity parameters:
   o Peak velocity:
      As mentioned above, peak velocity is the highest velocity value of the effective velocity trajectory.
      Peak velocity of specific parts of each vergence effective trajectory may also be calculated, including the peak velocity of the initial phasic component. However, it should be noted that peak velocity is clearly found during the initial phasic component and is thus equal to the peak velocity of the initial phasic component
   o Average velocity of the initial phasic component:
      This parameter is defined as the ratio between the amplitude and the duration of the initial phasic component.
   ∘ Total average velocity:
      This parameter is defined as the ratio between the total amplitude and the total duration, as defined above.

For saccades, to evaluate binocular coordination of saccades, a disconjugate signal (i.e., left eye - right eye, see Figure 3) may also be first calculated and a disconjugacy parameter may then be calculated, defined as the difference in saccade amplitude between the left and right eye signal may be calculated in the initial phasic component (referred to as "disconjugacy during saccade" parameter), or after the initial phasic component (referred to as "disconjugacy after saccade" or "post-saccadic drift disconjugacy"), in particular in the 80 ms component (referred to as "disconjugacy 80 ms after saccade" parameter), in the 160 ms component (referred to as "disconjugacy 160 ms after saccade" parameter).

Saccade (conjugate and disconjugate) parameters are preferably calculated separately for right and left saccades trajectories.

No matter which saccade parameter(s) is(are) calculated, the calculation may be made using any suitable software able to compute saccade effective trajectories from the recordings made by the eye tracker and calculate saccade parameters. Such software may preferably be the AIDEAL^{®} software disclosed in French patent application filed on May 14, 2020 under number FR2004768, the content of which is herein incorporated by reference.

### Preferred binocular parameters calculated in step c1)

The inventors showed that individuals suffering from vertigo have higher than normal latency and smaller amplitude during vergences (see Example 1). Therefore, preferred binocular parameters calculated in step c1) include latency and total amplitude of convergences and divergences during a vergence test, in particular latency and amplitude of divergences during a vergence test.

However, in order to provide a comprehensive assessment of binocular motricity of the individual, a more comprehensive combination of all available binocular parameters described above may preferably be calculated in step c1).

In particular, in addition to latency and amplitudes of convergences and divergences, binocular parameters described above may preferably be calculated in step c1) may further include:
- One or more other vergence parameter(s) during the vergence tests, such as another duration parameter than latency, another amplitude parameter than total amplitude, and/or one or more velocity parameter;
- One or more saccade parameter(s) during the saccade tests, such as one or more duration parameter, one or more amplitude parameter, and one or more velocity parameter;
- One or more vergence para parameter(s) meters during the combined tests, such as one or more duration parameter, one or more amplitude parameter, and one or more velocity parameter;
- One or more saccade parameter(s) during the combined tests, such as one or more duration parameter, one or more amplitude parameter, and one or more velocity parameter.
- One or more binocular parameters during the first vestibulo-ocular reflex (VOR) test at near and the second VOR test at far, including one or more of:
   o the conjugate ((left eye+right eye)/2) amplitude of eye movements from peak to peak (left to right to left), and
   o the disconjugate (left eye-right eye) amplitude of eye movements from peak to peak (left to right to left).

### Postural parameters

### Body sway parameters

Various postural parameters may be calculated from the body sway recorded by the device comprising three orthogonally mounted accelerometers in transverse, sagittal and coronal planes.

Preferably, the postural parameters calculated in step c1) comprise one or more of the following body sway parameters:
- Standardized area (expressed in mm²/s, and is calculated by dividing the area in mm² of the ellipse region containing 95% of the information points by the duration of the measurement),
- Root-Mean-Square of the Medio-Lateral Body Sway (RMS of M/L Body Sway in mm),
- Root-Mean-Square of the Antero-Posterior Body Sway (RMS of A/P Body Sway in mm),
- Root-Mean-Square of the Medio-Lateral velocity (RMS of M/L velocity in mm/s),
- Root-Mean-Square of the Antero-Posterior velocity (RMS of A/P velocity in mm/s), and
- Mean power frequency (Hz).

These parameters are extracted from the raw gravitational acceleration data in g (1 g ≈ 9,81 m/s²). After the data are high-pass filtered, velocity is calculated in the A/P and M/L direction by integration of the acceleration signal and displacement by integration of the velocity signal.

The first three parameters describe the stability while the last three concern mostly the energy used to stabilize the body.

### Head rotations parameters

The head acceleration signal is double integrated to obtain the head instantaneous position and head rotation: then the gaze signal is estimated, i.e. the head instantaneous position minus the eye instantaneous position (this method is standard, see Guitton D, Volle M. Gaze control in humans: eye-head coordination during orienting movements to targets within and beyond the oculomotor range. J Neurophysiol. 1987 Sep;58(3):427-59). The ratio of the amplitude of the conjugate eye movements ((left eye+right eye)/2) divided by the amplitude of head rotation, the so-called gain of the VOR is preferably also calculated.

The average amplitude of head rotation (left/right, or up/down) and its variability during the test is then calculated as well as the average gaze amplitude and its variability, as well as the average gain of the VOR and its variability.

### Preferred combinations of parameters calculated in step c1)

When the method for measuring binocular motricity and postural parameters in an individual suffering from vertigo according to the invention comprises only two vergence tests in step a1) (no additional saccade or combined tests), the parameters calculated in step c1) preferably comprise:
- Vergence parameters during the two vergence tests, comprising latency of convergences and divergences and total amplitude of convergences and divergences, optionally further comprising one or more other vergence parameter(s) during the two vergence tests;
- One or more binocular parameters during the first vestibulo-ocular reflex (VOR) test at near and the second VOR test at far, including one or more of the conjugate ((left eye+right eye)/2) amplitude of eye movements from peak to peak (left to right to left) and the disconjugate (left eye-right eye) amplitude of eye movements from peak to peak (left to right to left), and
- Postural parameters during the posture tests, comprising one or more (including all) of:
   - Body sway parameters for posture tests i) (vergence test in standing position), ii) and iii) (fixation tests at near and at far):
      ▪ Standardized area,
      ▪ Root-Mean-Square of the Medio-Lateral Body Sway,
      ▪ Root-Mean-Square of the Antero-Posterior Body Sway,
      ▪ Root-Mean-Square of the Medio-Lateral velocity,
      ▪ Root-Mean-Square of the Antero-Posterior velocity, and
      ▪ Mean power frequency ;
   - Head rotation parameters for posture tests iv) and v) (VOR tests at near and far):
      ▪ average amplitude of head rotation (left/right, or up/down) and its variability,
      ▪ average gaze amplitude and its variability, and
      ▪ average gain of the VOR and its variability.

When the method for measuring binocular motricity and postural parameters in an individual suffering from vertigo according to the invention further comprises two saccade tests in step a1), the parameters calculated in step c1) preferably comprise:
- Vergence parameters during the two vergence tests, comprising latency of convergences and divergences and total amplitude of convergences and divergences, optionally further comprising one or more other vergence parameter(s) during the two vergence tests;
- Saccade parameters during the two saccade tests, such as one or more duration parameter, one or more amplitude parameter, and one or more velocity parameter;
- One or more binocular parameters during the first vestibulo-ocular reflex (VOR) test at near and the second VOR test at far, including one or more of the conjugate ((left eye+right eye)/2) amplitude of eye movements from peak to peak (left to right to left) and the disconjugate (left eye-right eye) amplitude of eye movements from peak to peak (left to right to left), and
- Postural parameters during the posture tests, comprising one or more (including all) of:
   - Body sway parameters for posture tests i) (vergence test in standing position), ii) and iii) (fixation tests at near and at far):
      ▪ Standardized area,
      ▪ Root-Mean-Square of the Medio-Lateral Body Sway,
      ▪ Root-Mean-Square of the Antero-Posterior Body Sway,
      ▪ Root-Mean-Square of the Medio-Lateral velocity,
      ▪ Root-Mean-Square of the Antero-Posterior velocity, and
      ▪ Mean power frequency ;
   - Head rotation parameters for posture tests iv) and v) (VOR tests at near and far):
      ▪ average amplitude of head rotation (left/right, or up/down) and its variability,
      ▪ average gaze amplitude and its variability, and
      ▪ average gain of the VOR and its variability.

When the method for measuring binocular motricity and postural parameters in an individual suffering from vertigo according to the invention further comprises two combined tests in step a1), the parameters calculated in step c1) preferably comprise:
- Vergence parameters during the two vergence tests, comprising latency of convergences and divergences and total amplitude of convergences and divergences, optionally further comprising one or more other vergence parameter(s) during the two vergence tests;
- Vergence parameters during the two combined tests, such as one or more duration parameter, one or more amplitude parameter, and one or more velocity parameter;
- Saccade parameters during the two combined tests, such as one or more duration parameter, one or more amplitude parameter, and one or more velocity parameter;
- One or more binocular parameters during the first vestibulo-ocular reflex (VOR) test at near and the second VOR test at far, including one or more of the conjugate ((left eye+right eye)/2) amplitude of eye movements from peak to peak (left to right to left) and the disconjugate (left eye-right eye) amplitude of eye movements from peak to peak (left to right to left), and
- Postural parameters during the posture tests, comprising one or more (including all) of:
   - Body sway parameters for posture tests i) (vergence test in standing position), ii) and iii) (fixation tests at near and at far):
      ▪ Standardized area,
      ▪ Root-Mean-Square of the Medio-Lateral Body Sway,
      ▪ Root-Mean-Square of the Antero-Posterior Body Sway,
      ▪ Root-Mean-Square of the Medio-Lateral velocity,
      ▪ Root-Mean-Square of the Antero-Posterior velocity, and
      ▪ Mean power frequency ;
   - Head rotation parameters for posture tests iv) and v) (VOR tests at near and far):
      ▪ average amplitude of head rotation (left/right, or up/down) and its variability,
      ▪ average gaze amplitude and its variability, and
      ▪ average gain of the VOR and its variability.

When the method for measuring binocular motricity and postural parameters in an individual suffering from vertigo according to the invention further comprises two saccade tests and two combined tests in step a1),
the parameters calculated in step c1) preferably comprise:
- Vergence parameters during the two vergence tests, comprising latency of convergences and divergences and total amplitude of convergences and divergences, optionally further comprising one or more other vergence parameter(s) during the two vergence tests;
- Saccade parameters during the two saccade tests, such as one or more duration parameter, one or more amplitude parameter, and one or more velocity parameter;
- Vergence parameters during the two combined tests, such as one or more duration parameter, one or more amplitude parameter, and one or more velocity parameter;
- Saccade parameters during the two combined tests, such as one or more duration parameter, one or more amplitude parameter, and one or more velocity parameter; and
- Postural parameters during the posture tests, comprising one or more (including all) of:
   - Body sway parameters for posture tests i) (vergence test in standing position), ii) and iii) (fixation tests at near and at far):
      ▪ Standardized area,
      ▪ Root-Mean-Square of the Medio-Lateral Body Sway,
      ▪ Root-Mean-Square of the Antero-Posterior Body Sway,
      ▪ Root-Mean-Square of the Medio-Lateral velocity,
      ▪ Root-Mean-Square of the Antero-Posterior velocity, and
      ▪ Mean power frequency ;
   - Head rotation parameters for posture tests iv) and v) (VOR tests at near and far):
      ▪ average amplitude of head rotation (left/right, or up/down) and its variability,
      ▪ average gaze amplitude and its variability, and
      ▪ average gain of the VOR and its variability.

### Individual suffering from vertigo

The first method according to the invention for measuring binocular coordination and postural parameters may be performed for any individual suffering from vertigo.

In particular, the first method according to the invention may be performed either in patients with vertigo of organic origin (i.e. due to brain damage, generally involving hyporeflectivity, also referred to as "central vertigo") or in patients with vertigo of functional origin (i.e due to peripheral damage, generally involving hypersensitivity, also referred to as "peripheral vertigo", such as Meniere's disease and vestibular asthenopia). The first method according to the invention is particularly applicable to individuals with vertigo of functional origin, notably vestibular asthenopia and Meniere's disease.

### Method for detecting intrinsic ocular motor abnormalities and postural control abnormalities in an individual suffering from vertigo

The present invention also relates to a second a method for detecting intrinsic ocular motor abnormalities and postural control abnormalities in an individual suffering from vertigo, comprising:
a2) providing recordings of the eye movements of the left eye and the right eye of the individual during:
   - ocular motor tests comprising two vergence tests, and optionally two saccade tests and/or two combined tests, one being performed with the individual in seated position and the other with the individual in standing position,
   - posture tests comprising:
      iv. a first vestibulo-ocular reflex (VOR) test at near, consisting in turning the head left and right while keeping fixating a target at a first distance between 20 and 50 cm, and
      v. a second vestibulo-ocular reflex (VOR) test at far, consisting in turning the head left and right while keeping fixating a target at a second distance between 100 and 200 cm
b2) providing recordings of:
   - body sway of the individual during posture tests comprising:
      i) the vergence test, and optionally the saccade test and/or the combined test, performed in standing position of step a2),
      ii) a first fixation test at near, consisting in fixating a target for 20 to 40 seconds, preferably 30 seconds, at a first distance between 20 and 50 cm,
      iii) a second fixation test at far, consisting in fixating a target for 20 to 40 seconds, preferably 30 seconds, at a second distance between 100 and 200 cm,
   - head rotations of the individual during posture tests comprising:
      iv) a first vestibulo-ocular reflex (VOR) test at near, consisting in turning the head left and right while keeping fixating a target at a first distance between 20 and 50 cm, and
      v) a second vestibulo-ocular reflex (VOR) test at far, consisting in turning the head left and right while keeping fixating a target at a second distance between 100 and 200 cm,
c2) calculating, using a digital processing device, the value(s) of one or more binocular coordination parameter from the records provided in step a2) and the value(s) of one or more postural parameter(s) from the records provided in step b2);
d2) comparing the value(s) calculated in step c2) to one or more reference value(s) obtained in one or more individual(s) not suffering from vertigo;
e2) concluding to the presence of intrinsic ocular motor abnormalities in the individual suffering from vertigo if one or more binocular coordination parameter calculated in step c2) is significantly different from the one or more reference value(s) obtained in one or more individual(s) not suffering from vertigo; and
f2) concluding to the presence of postural control abnormalities in the individual suffering from vertigo if one or more postural parameter(s) calculated in step c2) is significantly different from the one or more reference value(s) obtained in one or more individual(s) not suffering from vertigo;

wherein the two vergence tests from which the recordings are provided in step a2) have been conducted using a binocular motor stimulation device configured to specifically and physically stimulate vergences and saccades, comprising visual or audiovisual targets placed in real space at eccentricities and depths of a surface;
wherein each of the two vergence tests which recordings are provided comprised 30 to 50 trials of:
   - visually and/or audiovisually stimulating the individual at a point located at the eyes level, in the central axis between the left eye and the right eye, at a first distance, and
   - visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved.

### Step a2): providing recordings of the eye movements of the left eye and the right eye of the individual

The method for detecting intrinsic ocular motor abnormalities and postural control abnormalities in an individual suffering from vertigo according to the invention is based on previously obtained recordings of the eye movements of the left eye and the right eye of the individual during:
- ocular motor tests comprising two vergence tests, and optionally two saccade tests and/or two combined tests, one being performed with the individual in seated position and the other with the individual in standing position,
- posture tests comprising:
   iv. a first vestibulo-ocular reflex (VOR) test at near, consisting in turning the head left and right while keeping fixating a target at a first distance between 20 and 50 cm, and
   v. a second vestibulo-ocular reflex (VOR) test at far, consisting in turning the head left and right while keeping fixating a target at a second distance between 100 and 200 cm

Such recordings have been made using an eye tracker as defined above for the first method for measuring binocular coordination and postural parameters according to the invention.

The vergence tests, optional saccade tests, optional combined tests and posture tests to which the individual suffering from vertigo has been submitted for obtaining the recordings provided in step a2) have the same general and preferred features and combinations of features as those disclosed above for the first method for measuring binocular coordination and postural parameters according to the invention.

### Step b2): providing recordings of body sway or head rotations of the individual during posture tests

The method for detecting intrinsic ocular motor abnormalities and postural control abnormalities in an individual suffering from vertigo according to the invention is also based on previously obtained recordings of
- body sway of the individual during posture tests comprising:
   i) the vergence test, and optionally the saccade test and/or the combined test, performed in standing position of step a2),
   ii) a first fixation test at near, consisting in fixating a target for 30 seconds at a first distance between 20 and 50 cm, and
   iii) a second fixation test at far, consisting in fixating a target at a second distance between 100 and 200 cm; and
- head rotations of the individual during posture tests comprising:
   iv) a first vestibulo-ocular reflex (VOR) test at near, consisting in turning the head left and right while keeping fixating a target at a first distance between 20 and 50 cm, and
   v) a second vestibulo-ocular reflex (VOR) test at far, consisting in turning the head left and right while keeping fixating a target at a second distance between 100 and 200 cm;
as disclosed above for the first method for measuring binocular coordination and postural parameters according to the invention.

In particular, the posture tests have the same general and preferred features and combinations of features as those disclosed above for the first method for measuring binocular coordination and postural parameters according to the invention.

### Step c2): calculating, using a digital processing device, the value(s) of one or more binocular coordination parameter from the records provided in step a2) and the value(s) of one or more postural parameter(s) from the records provided in step b2)

The binocular coordination parameter(s) calculated in step c2) of the second method for detecting intrinsic ocular motor abnormalities and postural control abnormalities in an individual suffering from vertigo according to the invention also have the same general and preferred features and combinations of features as those disclosed above for step c1) of the first method for measuring binocular coordination and postural parameters according to the invention.

### Step d2): comparing the value(s) calculated in step c2) to one or more reference value(s) obtained in one or more individual(s) not suffering from vertigo

In order to detect intrinsic ocular motor abnormalities in the individual suffering from vertigo, the value(s) of binocular coordination parameter(s) calculated in step c2) are compared with corresponding reference values obtained when submitting one or more individual(s) not suffering from vertigo to the same ocular motor tests or with reference values obtained when submitting one or more individual(s) suffering from vertigo to the same ocular motor tests.

In order to detect postural control abnormalities in the individual suffering from vertigo, the value(s) of postural parameters calculated in step c2) are compared with corresponding reference values obtained when submitting one or more individual(s) not suffering from vertigo to the same posture test or with reference values obtained when submitting one or more individual(s) suffering from vertigo to the same posture test.

### Step e2): concluding to the presence of intrinsic ocular motor abnormalities

Based on the comparison made in step d2), it may be concluded to the presence or absence of intrinsic ocular motor abnormalities in the individual suffering from vertigo. In particular, it is concluded to the presence of intrinsic ocular motor abnormalities in the individual suffering from vertigo if at least one binocular coordination parameter calculated in step c2) is significantly different from the corresponding reference value(s) obtained in one or more individual(s) not suffering from vertigo.

In contrast, it is concluded to the absence of intrinsic ocular motor abnormalities in the individual suffering from vertigo if the binocular coordination parameter(s) calculated in step c2) are not significantly different from the corresponding reference value(s) obtained in one or more individual(s) not suffering from vertigo.

Notably, reference normal ranges or average values of binocular coordination parameter(s) may be used in step e2). In this case, it may be concluded to:
- absence of intrinsic ocular motor abnormalities in the individual suffering from vertigo if all binocular coordination parameters calculated in step c2) are inside the corresponding reference normal range or are less than 20% above or below the corresponding reference average value in healthy individuals, and
- presence of intrinsic ocular motor abnormalities in the individual suffering from vertigo if at least one binocular coordination parameter calculated in step c2) is outside the corresponding reference normal range or at least 20% above or below the corresponding reference average value in healthy individuals.

While a conclusion may be drawn based on direct comparison, one by one, of the value(s) of binocular coordination parameter(s) calculated in step c2) with corresponding reference values obtained when submitting one or more individual(s) not suffering from vertigo to the same vergence and optional saccade and/or combined tests or with reference values obtained when submitting one or more individual(s) suffering from vertigo to the same vergence and optional saccade and/or combined tests, it is also possible to use an algorithm (also referred to as a classifier) that will classify the individual as suffering or not suffering from vertigo based on the value(s) of binocular coordination parameter(s) calculated in step c2).

The classifier has been previously trained based on the value(s) of binocular coordination parameter(s) calculated in step c2) obtained from a cohort comprising both individuals suffering from vertigo and individuals not suffering from vertigo.

Any suitable classifier may be used, including both linear and non-linear classifiers. Examples of classifiers that maybe used include logistic regression, Support Vector Machine (SVM), Ensembing, GaussianProcessClassifier, random-forest, KNeighborsClassifier, AdaBoost, BernoulliNB, Linear discriminant analysis, GaussianNB, Decision Tree, Quadratic discriminant analysis and Multilayer perceptron.

Depending if intrinsic ocular motor abnormalities are or not detected in an individual suffering from vertigo using the method according to the invention, different types of management of the individual may be decided.

### Step f2): concluding to the presence of postural control abnormalities

Based on the comparison made in step d2), it may be concluded to the presence or absence of postural control abnormalities in the individual suffering from vertigo.

In particular, it is concluded to the presence of postural control abnormalities in the individual suffering from vertigo if at least one postural parameter calculated in step c2) is significantly different from the corresponding reference value(s) obtained in one or more individual(s) not suffering from vertigo.

In contrast, it is concluded to the absence of postural control abnormalities in the individual suffering from vertigo if the postural parameter(s) calculated in step c2) are not significantly different from the corresponding reference value(s) obtained in one or more individual(s) not suffering from vertigo.

Notably, reference normal ranges or average values of postural parameter(s) may be used in step e2). In this case, it may be concluded to:
- absence of postural control abnormalities in the individual suffering from vertigo if all postural parameters calculated in step c2) are inside the corresponding reference normal range or are less than 20% above or below the corresponding reference average value in healthy individuals, and
- presence of postural control abnormalities in the individual suffering from vertigo if at least one postural parameter calculated in step c2) is outside the corresponding reference normal range or at least 20% above or below the corresponding reference average value in healthy individuals.

While a conclusion may be drawn based on direct comparison, one by one, of the value(s) of postural parameter(s) calculated in step c2) with corresponding reference values obtained when submitting one or more individual(s) not suffering from vertigo to the same posture tests (body sway or head rotations) or with reference values obtained when submitting one or more individual(s) suffering from vertigo to the same posture tests (body sway or head rotations), it is also possible to use an algorithm (also referred to as a classifier) that will classify the individual as suffering or not suffering from vertigo based on the value(s) of postural parameter(s) calculated in step c2).

The classifier has been previously trained based on the value(s) of postural parameter(s) calculated in step c2) obtained from a cohort comprising both individuals suffering from vertigo and individuals not suffering from vertigo.

Any suitable classifier may be used, including both linear and non-linear classifiers. Examples of classifiers that maybe used include logistic regression, Support Vector Machine (SVM), Ensembing, GaussianProcessClassifier, random-forest, KNeighborsClassifier, AdaBoost, BernoulliNB, Linear discriminant analysis, GaussianNB, Decision Tree, Quadratic discriminant analysis and Multilayer perceptron.

Depending if postural control abnormalities are or not detected in an individual suffering from vertigo using the method according to the invention, different types of management of the individual may be decided.

### Individual suffering from vertigo

The second method according to the invention for detecting intrinsic ocular motor abnormalities and postural control abnormalities in an individual suffering from vertigo may be performed for any individual suffering from vertigo, as described above for the first method according to the invention for measuring binocular motricity and postural parameters in an individual suffering from vertigo.

In particular, the second method according to the invention may be performed either in patients with vertigo of organic origin or in patients with vertigo of functional origin. The second method according to the invention is particularly applicable to individuals with vertigo of functional origin, notably vestibular asthenopia and Meniere's disease.

### Method for improving binocular coordination and postural control in an individual suffering from vertigo

The inventors have found that most individuals suffering from vertigo have intrinsic vergence abnormalities, and that these abnormalities may at least partly be corrected by submitting the individual to vergence training sessions.

In a third aspect, the present invention thus also relates to a third a method for improving binocular coordination and postural control in an individual suffering from vertigo, comprising:
a3) submitting the individual to ocular motor tests comprising two vergence tests, and optionally two saccade tests and/or two combined tests, one being performed with the individual in seated position and the other with the individual in standing position, and recording the eye movements of the left eye and the right eye of the individual during the two vergence tests, and optionally during the two saccade tests and/or two combined tests;
b3) submitting the individual to posture tests comprising:
   i) the vergence test, and optionally the saccade test and/or the combined test, performed in standing position of step a1),
   ii) a first fixation test at near, consisting in fixating a target for 20 to 40 seconds, preferably 30 seconds, at a first distance between 20 and 50 cm,
   iii) a second fixation test at far, consisting in fixating a target for 20 to 40 seconds, preferably 30 seconds, at a second distance between 100 and 200 cm,
   iv) a first vestibulo-ocular reflex (VOR) test at near, consisting in turning the head left and right while keeping fixating a target at a first distance between 20 and 50 cm, and
   v) a second vestibulo-ocular reflex (VOR) test at far, consisting in turning the head left and right while keeping fixating a target at a second distance between 100 and 200 cm,
   and recording the eye movements of the left eye and the right eye of the individual in posture tests i), iv) and v), recording body sway of the individual during the posture tests i) to iii); and recording head rotations during posture tests iv) and v);
c3) calculating, using a digital processing device, the value of one or more binocular coordination parameter from the records obtained in step a3) and the value of a one or more postural parameter from the records obtained in step b3);
d3) comparing the value(s) calculated in step c3) to one or more reference value(s) obtained in one or more individual(s) not suffering from vertigo;
e3) concluding to the presence of intrinsic ocular motor abnormalities in the individual suffering from vertigo if one or more binocular coordination parameter calculated in step c3) is significantly different from the one or more reference value(s) obtained in one or more individual(s) not suffering from vertigo; and
f3) concluding to the presence of postural control abnormalities in the individual suffering from vertigo if one or more postural parameter(s) calculated in step c3) is significantly different from the one or more reference value(s) obtained in one or more individual(s) not suffering from vertigo;
g3) submitting the individual to vergence training sessions once or twice a week between 2 to 6 weeks; and
h3) repeating steps a3) to f3), and optionally step g3), until some improvement in the intrinsic ocular motor abnormalities of the individual suffering from vertigo is found;

wherein the vergence tests and the vergence training sessions, and optionally the saccade tests and/or the combined tests, are conducted using a binocular motor stimulation device configured to specifically and physically stimulate vergences and saccades, comprising visual or audiovisual targets placed in real space at eccentricities and depths of a surface;
wherein each of the two vergence tests comprises 30 to 50 trials of:
   - visually and/or audiovisually stimulating the individual at a point located at the eyes level, in the central axis between the left eye and the right eye, at a first distance, and
   - visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved.

### Steps a3) to f3)

Steps a3) to c3) of the third method according to the invention (for improving binocular coordination and postural control in an individual suffering from vertigo) are the same as steps a1) to c1) of the first method according to the invention (for measuring binocular coordination and postural parameters in an individual suffering from vertigo) as disclosed above.

Similarly, steps d3) to f3) of the third method according to the invention are the same as steps d2) to f2) of the second method according to the invention (for detecting intrinsic ocular motor abnormalities and postural control abnormalities in an individual suffering from vertigo) as disclosed above.

These steps thus have the same general and preferred features and combinations of features as those disclosed above in the corresponding method.

In particular, the ocular motor texts of step a3) optionally and preferably comprise, in addition to the two vergence tests, two saccade tests and/or two combined tests, more preferably ocular motor texts of step a3) preferably comprise two vergence tests and two saccade tests, or two vergence tests, two saccade tests and two combined tests, as disclosed above for the first method according to the invention.

### Step g3): vergence training sessions

In step g3), the individual suffering from vertigo is submitted to vergence training sessions once or twice a week between 2 to 6 weeks, preferably 3 to 5 weeks, such as 3, 4 or 5 weeks. Preferably, the individual is submitted to at least 4 (or at least 5, at least 6, at least 7, at least 8, such as 4, 5 6, 7, 8, 9 or 10) vergence training sessions (also referred to as "vergence rehabilitation sessions" or "vergence rehabilitation training sessions"). The period between two successive vergence training sessions is preferably of 2 to 7 days, such as 2, 3, 4, 5, 6 or 7 days. The period between different successive vergence training sessions may vary, depending on the availabilities of the individual and visual health professional. However, the vergence training sessions are preferably regularly spaced.

Each vergence training session comprises 1 or 2 repetitions of 1 or 2 block(s) of divergence followed by 3 to 5 blocks of convergence. For instance, a vergence training session may comprise:
- 1 block of divergence, followed by 4 blocks of convergence. When each block contains 40 trials, each block is about 2-3 minutes and such a training session has a duration of about 15 minutes.
- 2 blocks of divergence, 3 blocks of convergence, 2 blocks of divergence, 3 blocks of convergence, and a final block of divergence. When each block contains 40 trials, each block is about 2-3 minutes and such a training session has a duration of about 35 minutes.

While all vergence training sessions to which the individual suffering from vertigo is submitted in step g3) may be identical, it is also possible for the visual health professional to change the precise content of vergence training sessions during step g3).

Each block of a vergence training session comprises 30 to 50 trials, preferably an even number of trials between 30 and 50, more preferably between 36 and 44 trials, such as 36, 38, 40, 42 or 44 trials, in particular 40 trials.

The trials of the vergence training or rehabilitation procedure are not the same as in the vergence testing procedure.

After an initial fixation of an LED presented at various depths along the central line the first target LED stimulus is only activated for a short period (between 150 and 200 ms); following this period, another target stimulus is activated for a period of 800 to 1800 ms. preferably 1300 ms. The vergence rehabilitation training protocol based on sequences of the double step type is more drastic and very effective because it triggers the implementation by the central nervous system of a new adaptive control (generation of a motor command in response to the final stimulus and not in response to the initial transitory stimulus). The technique can be particularly useful as it stimulates natural ocular motor plasticity. During convergence training blocks the second target is closer to the individual's eyes that the first one, and the opposite during the divergence training trials.

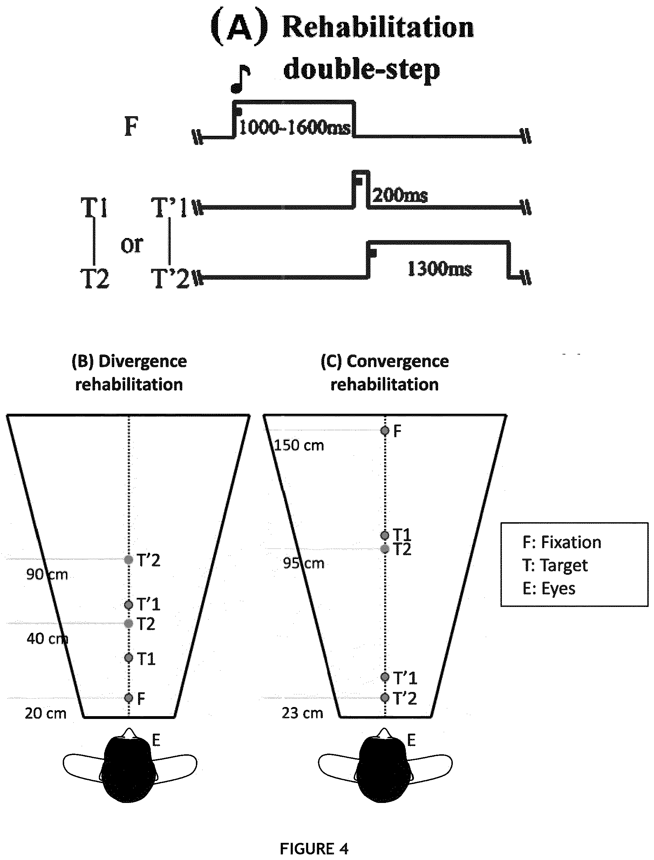

Each trial of each block of a vergence training session comprises:
- visually and/or audiovisually stimulating the individual suffering from vertigo at a first point located at the eyes level, in the central axis between the left eye and the right eye for a period varying from 1000 to 1600 ms, at a first distance (for instance illustrated by point F in Figures 4B and 4C),
- visually and/or audiovisually stimulating the individual suffering from vertigo at a second point located at the eyes level, in the central axis between the left eye and the right eye for a period of 150 to 200 ms, preferably 200 ms, at a second distance calling for a convergence movement in convergence blocks (for instance illustrated by point T1 or T1' in Figure 4C) or a divergence movement in divergence blocks (for instance illustrated by point T1 or T1' in Figure 4B), and
- visually and/or audiovisually stimulating the individual suffering from vertigo at a third point located at the eyes level, in the central axis between the left eye and the right eye for a period varying from 800 to 1800 ms, preferably 1000 to 1600 ms, in particular 1300 ms, at a third distance calling for a further convergence movement in convergence blocks (for instance illustrated by point T2 or T2' in Figure 4C) or a further divergence movement in divergence blocks (for instance illustrated by point T2 or T2' in Figure 4B).

This type of protocol is referred to as a vergence double-step protocol, and is based on the fact that the target steps to a second position before the vergence movement completion. Given that the vergence latency is between 160 and 250 ms, and vergence execution lasts between 350 and 550 ms, it is almost certain that the second step of the target occurred before the initial vergence eye movement has been made. This type of protocol is designed to expose the visuo-motor system to an error that cannot be corrected online and leads to adaptive readjustment of the gain of the motor control.

Depending on the results of the vergence tests performed in seated and standing position, the vergence training sessions may be performed with the individual in seated position, with the individual in standing position, or with the individual alternating the seated and standing positions.

Preferably, the vergence training sessions are performed with the individual alternating the seated and standing positions. In this case, some blocks of a vergence training session are performed in seated position while other blocks are performed in standing position. The relative proportion of blocks performed in seated position and in standing position may be selected by the visual health professional depending on the results of the vergence tests performed in seated and standing position.

In general, more blocks will be done in the seated position if the patient has weaker initial tests in the seated position and vice versa.

### Step h3)

In step h3), steps a3) to f3) (functional exploration), and optionally step g3) (rehabilitation), are repeated until some improvement in the intrinsic ocular motor abnormalities and/or postural control abnormalities of the individual suffering from vertigo is found.

In many cases, provided that the individual has been submitted to a sufficient number of regularly spaced vergence training sessions, step g3) (rehabilitation) will not have to be repeated.

However, in some cases, in particular when the intrinsic ocular abnormalities and/or postural control abnormalities are severe or when numerous abnormalities have been detected in steps e3) and f3), the visual health professional may choose to submit the individual to a limited number of vergence training sessions (in the range defined above in step g3)) and then repeat steps a3) to f3) (functional exploration) in order to assess improvements and determine how many further vergence training sessions may be needed when repeating step g3). In any case, functional exploration (steps a3) to f3)) is preferably performed systematically after step g3) (rehabilitation).

### Individual suffering from vertigo

The third method according to the invention for improving binocular coordination and postural control in an individual suffering from vertigo may be performed for any individual suffering from vertigo, as described above for the first method according to the invention for measuring binocular motricity and postural parameters in an individual suffering from vertigo.

In particular, the third method according to the invention may be performed either in patients with vertigo of organic origin or in patients with vertigo of functional origin.

The third method according to the invention is particularly applicable to individuals with vertigo of functional origin, notably vestibular asthenopia and Meniere's disease, since particular benefits of vergence rehabilitation sessions has been observed in these patients (see Example 1).

The following examples merely intend to illustrate the present invention.

### EXAMPLES

### Example 1: Anomalies of vergence in patients with vertigo: diagnosis and neuro-rehabilitation with REMOBI

### Patients, Materials and Methods

### Patients

Fourteen vertiginous patients participated in the study. The "vertigo" symptom was the essential criterion of inclusion. These patients were initially assessed on the ENT plan and underwent a complete orthoptic examination. Vestibular function was assessed by videonystagmoscopy (VNS) in complete darkness. None of these patients were in acute attacks of vertigo. In other words, none of them had spontaneous nystagmus that could be observed with bare eyes. The ocular fixation index was therefore correct, that is to say that the visual fixation completely inhibited any spontaneous vestibular nystagmus observed under VNS.

### REMOBI, vergence test and rehabilitation

In order to objectively evaluate the presence of abnormalities of vergences, we used the device REMOBI. REMOBI (US Patent 8851669), is a trapezoid-shaped horizontal device, equipped with visual-acoustic elements (diodes and buzzers) and embedded software. REMOBI is a device that contains specific and different algorithms, on the one hand for the assessment (diagnosis of anomalies of vergences), on the other hand, for their neuro-rehabilitation of vergence movements. The patient's eye movements were recorded in this study with the EyeSeeCam video-oculography, binocular with a frame rate of each eye at 220 Hz (see eyeseecam.com, Munich).

*Test for the assessment of vergences:* the vergence movements were tested with the following paradigm: at each test a fixation diode was presented in the median plane at 40cm of the subject during 1500ms, preceded by a short beep which served as a warning attentional; then a second diode appeared 1200 to 1800ms after the beginning of the first presentation, is located at 1.50m from the subject, or located 20cm from the subject. In the first case, a divergence movement had to be made a diode to fix further away.

In the second case, a convergence movement had to be made to fix a diode closer (see **Figure 1C****).** This vergence test consisted of 40 randomly interpolated trials containing 20 convergence and 20 divergence trials.

*Vergence rehabilitation protocol:* for the rehabilitation of vergence, we used another embedded algorithm at REMOBI, the so-called Vergence protocol in double step, detailed in the study by Kapoula, Z. et al. Transl Vis Sci Technol 5, 8, doi:10.1167/tvst.5.2.8 (2016). During this protocol, the subject must make a vergence movement towards the first target diode. This target, however, is presented only for a very short time and is replaced by a second target, even closer to convergence training and even more distant for divergent training, forcing the subject to quickly reprogram the initial movement (see **Figure 4**). The results brought by Kapoula, Z. et al. Transl Vis Sci Technol 5, 8, doi:10.1167/tvst.5.2.8 (2016) in students with vergence abnormalities demonstrated the efficiency of this method; indeed, after 5 sessions of 35 min we confirmed an amplification and acceleration of convergences and divergences, lasting 18 months later. In this study, 4 rehabilitation sessions were performed with the REMOBI device by the service orthoptist. The sessions were held weekly. Eye movements were not recorded during these sessions, each lasting 20 minutes.

The rehabilitation of the vergences was done by alternating either the sitting position or the standing position. Each time the REMOBI device was placed at eye level. In the standing position, the person leads the synergy between vergences and postural balance in a more active way, which results in a real reeducation combining the gaze and the vestibular and postural function.

The vergence test (assessment) presented above, including oculomotor recording by video-oculography, was performed before the rehabilitation and 1 month after the end of the 4 rehabilitation sessions.

### Posture test

The body sway was measured with the small DynaPort MiniMod ^{®} (McRoberts B.V. The Hague, The Netherlands) device (74 g) equipped with three intransverse, sagittal and coronal orthogonally mounted accelerometers (AXXL202, AnalogueDevices, Norwood MA, USA), placed on a belt at the lumbosacral level and near the center of mass of the body was evaluated. The frequency of sampling is set at 100 Hz. This device is known to be easy to use in the elderly, during various activities such as cycling, sitting, standing, and recently during quiet upright.

Postural parameters: We measured the following parameters: (1) standardized area (in mm2/s), area of the ellipse region containing 95% of the information points in mm², divided by the duration of the measurement, (2) the Root-Mean-square of the Medio-Lateral Body Sway (RMS of M / L in mm), (3) the Root-Mean-Square of the Antero-Posterior Body Sway (RMS of A / P inmm), (4) the Root-Mean-Square of the Medio-Lateral velocity (in mm/s), (5) the Root-Mean-Square of the Antero-Posterior velocity (in mm/s). These parameters are obtained in g from the raw information of gravity acceleration. After the information is filtered high-pass, the velocity is calculated in the direction of A/P and M/L by means of the acceleration signal integration and displacement by means of the velocity signal integration.

### Data Analysis

Data recorded with the Pupil Labs eye tracker were analyzed with AIDEAL, software developed in the IRIS laboratory (see French patent application filed on May 14, 2020 under number FR2004768, DSO2020003510, 2 mars 2020). The vergence signal was derived by calculating the difference between the two eyes from the individual calibrated eye position signals (i.e., left eye - right eye). The beginning and end of the vergence movements were defined as the time point when the eye velocity exceeded or dropped below 5° /s: these criteria are standard and were applied automatically by the AIDEAL software; the program estimated the initial phasic component as the amplitude between these two initial points. It also calculated the amplitude change during the subsequent 80 ms and 160 ms. The total amplitude was calculated as the sum of the amplitude of the initial phasic component plus the 160 ms component. The total duration was calculated as the duration of the phasic component plus the subsequent 160 ms.

For saccade analysis, AIDEAL treated the conjugate signal, e.g. the L+R eye position/2. The onset and the offset of the saccade were defined as the time points where the velocity went above or below 10% of the peak velocity; practically, this corresponded to values above or below 40°/s (as the peak velocity of 20° saccades is typically above 400° /s). The total average velocity was defined as the ratio of total amplitude in degrees divided by time in seconds. To evaluate binocular coordination of saccades, or the disconjugacy during saccadic movements, the difference in amplitude between the left and the right eye signal was calculated. The disconjugate drift, or the difference in drift amplitude during the first 80 or 160 ms of fixation, was calculated. These calculations are standard and have been used in previous experiments (Kapoula, Z. et al. Transl Vis Sci Technol 5, 8, doi:10.1167/tvst.5.2.8 (2016) ; Morize, A., et al. Invest Ophthalmol Vis Sci 58, 329-342, doi:10.1167/iovs.16-19837 (2017)).

Trials with blinks or other artifacts were discarded automatically by AIDEAL.

### Results

### Patients characteristics

**Table 1 below present patients characteristics:**

| | Gender and age | Main complaint | Age of vertigo symptoms | Fusion amplitudes | PPC (cm) | Vestibular state |
|---|---|---|---|---|---|---|
| 1 | M 76 | Positional vertigo | 3 weeks | strabic | 8 | Hypersensitivity |
| 2 | F 38 | Vestibular Athenopia | 5 weeks | C'25 C18 D'10 D2 | 5 | Hypersensitivity |
| 3 | M 79 | Vertigo and instability | 3 years | C'18 C8 D'12 D4 | 7 | Spontaneous right |
| 4 | M 58 | Vertigo and diplopie | 2 weeks | C'30 C20 D'12 D4 | 10 | Left hyporeflectivity |
| 5 | F 55 | Vestibular Athenopia | 6 weeks | C'35 C8 D'12 D4 | 8 | Hypersensitivity |
| 6 | F 63 | Discreet vertigo | 8 years | C'20 C8 D'12 D4 | 7 | Right hyporeflectivity |
| 7 | F 52 | Vestibular Athenopia | 2 months | C'35 C18 D'12 D10 | 5 | Nys. Sp. Vert. Inf. idiop. |
| 8 | M 64 | Positional vertigo | 10 days | C'20 C16 D'10 D2 | 5 | Hypersensitivity |
| 9 | M 40 | Discreet vertigo | 3 weeks | C'14 C8 D'10 D2 | 5 | Left hyporeflectivity |
| 10 | F 53 | Discreet vertigo | 15 days | C'30 C4 D'6 D2 | 5 | Hypersensitivity |
| 11 | F 33 | Vestibular Athenopia | 3 months | C'18 C18 D'12 D4 | 3 | Hypersensitivity |
| 12 | F 48 | Discreet vertigo | 5 months | C'35 C14 D'8 D2 | 3 | Right hyporeflectivity |
| 13 | M 61 | Chronic vertigo | 4 years | C'30 C4 D'6 D4 | 15 | Left hyporeflectivity |
| 14 | M 65 | Chronic vertigo | 8 years | C'0 C10 D'10 D10 | 7 | Right hyporeflectivity |

Table 1. Patients characteristics. Among the patient complaints, this table lists four types of vertiginous symptoms: Positional vertigo (vertigo triggered and felt during a supine position), Vestibular Athenopia (Set of relatively permanent visual and movement-related symptoms), Discreet vertigo (brief, episodic vertigo, perceived several times a day), and Chronic vertigo (recurrent vertigo with a rotational nature and evolving by seizures). The vestibular state gathers the observations made under videonystagmoscopy, the eye being filmed in the dark, under infrared camera. It indicates either the objective characteristics of an asymmetry of the vestibular balance or, in the absence of objective signs, a subjective notion of sensory discomfort related to movement: the presence of spontaneous nystagmus indicates an uncompensated vestibular state, and hyporeflectivity is the residual sign of vestibular hypofunction observable during stimulation during the rotational pulse test protocol.

### Examples of Eye Movement Results:

**Figures 5A and 5B** show the trajectories of the vergences of patient 5. Clinically, this patient complained of sensitivity to optic flow generating nausea appeared a few weeks before the first consultation, impressions of instability fleeting, of feeling of permanent flutter and discomfort in rapid movements of the head and eyes.

**Figure 5A** shows the trajectories of its convergences (traced upwards) and the trajectories of its divergences (traced downwards) during the 2.5 min of vergence test performed before rehabilitation. The bold lines indicate the position of the respective target that the eyes should reach. This figure shows a strong hypometry especially for convergence.

**Figure 5B** shows the trajectories of the patient 1 month after the 4 rehabilitation sessions. There is a significant acceleration and amplification of the convergence, the movement initially the most deficient. For divergence, there is a reduction in latency. Subjectively, the patient reported greater ease of visual exploration of her environment, a greater sense of stability, and increased confidence when walking.

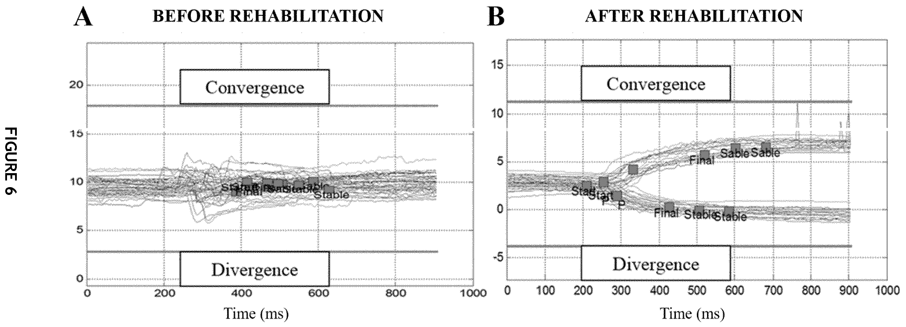

**Figure 6A** presents the results of patient 14, suffering from an old and well documented left Meniere's disease. He had undergone classical vestibular rehabilitation (consisting of 9 rotary chair sessions). This rehabilitation was beneficial and avoided surgery with its labyrinthine risk. However, episodically, small attacks of low intensity and well tolerated by the patient reappeared; indeed, the periodically reproduced vestibular examination showed a state of marked left deficit with right directional gravity.

Interestingly, we see in **Figure 6A** a paucity of vergences. The patient tries to converge by performing earlier jerky movements, of low amplitude, reaching neither the target of the convergence (bold line at high) nor the target of the divergence (bold line down). We then conclude in the existence of a major deficit of all vergences for this patient suffering from several years of Meniere's disease.

A major improvement in vergence trajectories is observed after rehabilitation **(****Figure 6B****).**

Vergence problems exist in 39% of the population, but in the presence of vestibular pathology, as in this study, nearly 100% of patients have problems with vergence. Fifty percent of the patients, 8 of the 14 patients studied, experienced an improvement in the quality of their vergences: in only four REMOBI neuro-rehabilitation sessions, the latency times decreased, the amplitudes or the speed increased, the movements became more regular and the saccade-vergence couple improved. Patients report better comfort in their relationship to space and their ability to move. The benefits were particularly significant in patients with vestibular asthenopia and Meniere's disease.

### Eye Movement Results

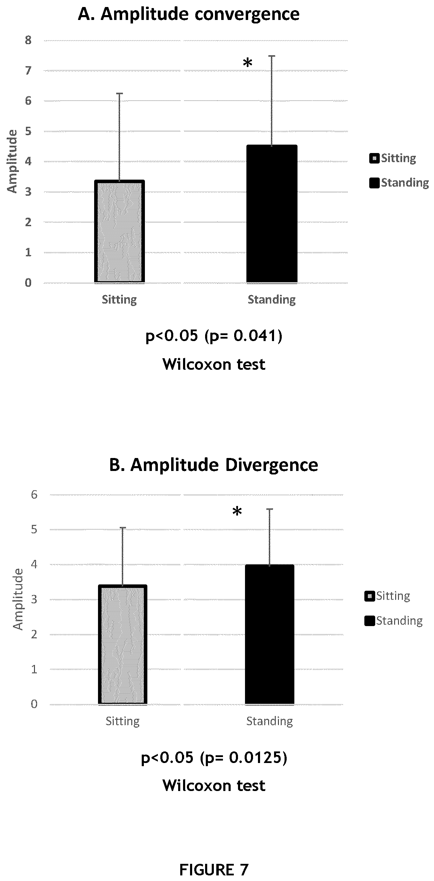

Observation of all subjects indicates a significant increase of the amplitude of divergence or convergence in the standing position compared to the seated condition (see **Figures 7A & 7B****).**

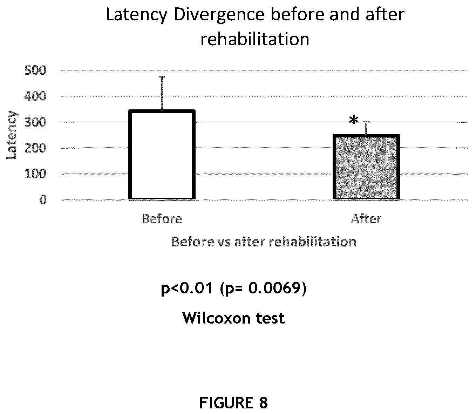

Also, there was a statistically significant difference in the latency of divergence when comparing the results of before and after rehabilitation (p<0.01, see **Figure 8****).**

### Analysis of a movement according to origin of vertigo:

In the following analysis we separated patients with functionally origin of vertigo from all the others whose vertigo symptoms were related to organic results. These additional analyses indicated significant differences that differed from the two groups.

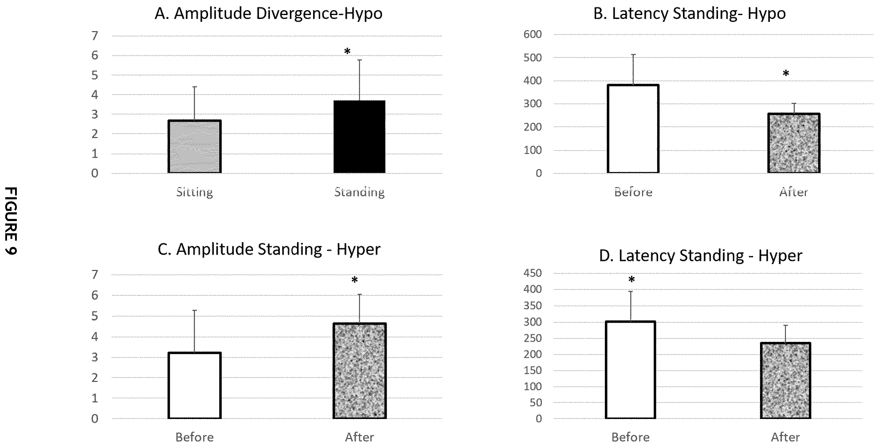

When considering the data for the patients with organic origin of the vertigo there is a statistically significant higher amplitude of divergence in standing than the seated condition (p = 0.0277, see **Figure 9A).** Also we find a significantly lower latency of the divergence in the post rehabilitation testing in standing condition relating to the pre rehabilitation testing (p = 0.0277, see **Figure 9B).**

For the subjects with the functional disorder that is Hyper Sensibility, there is a statistically significant higher amplitude of the divergence in standing in the post rehabilitation testing when compared to the pre rehabilitation testing (p = 0.0277, see **Figure 9C).**

Also the latency of divergence in standing condition decreased significantly in the post rehabilitation condition compared to the pre rehabilitation testing (p = 0.046, see **Figure 9D****).**

### Results on posture:

Unfortunately, for technical issues, postural measurements were done only during pre rehabilitation tests.

Considering all patient's they did not show any significant results.

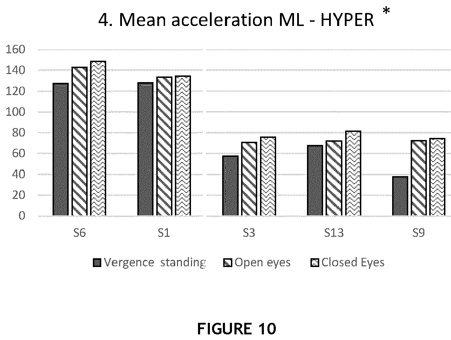

Considering the two groups in contrast:
- For the hypersensitivity group (or organic group), on observation one observes that the activation of the medio lateral body sway was statistically significant when actively performing vergence eye movements compared to when fixating near or far (see **Figure 10****)**
- Hypo group with organic origin of vertigo patients was not significantly different in posture between conditions, but there was a significant correlation between posture parameter and the eye movements.

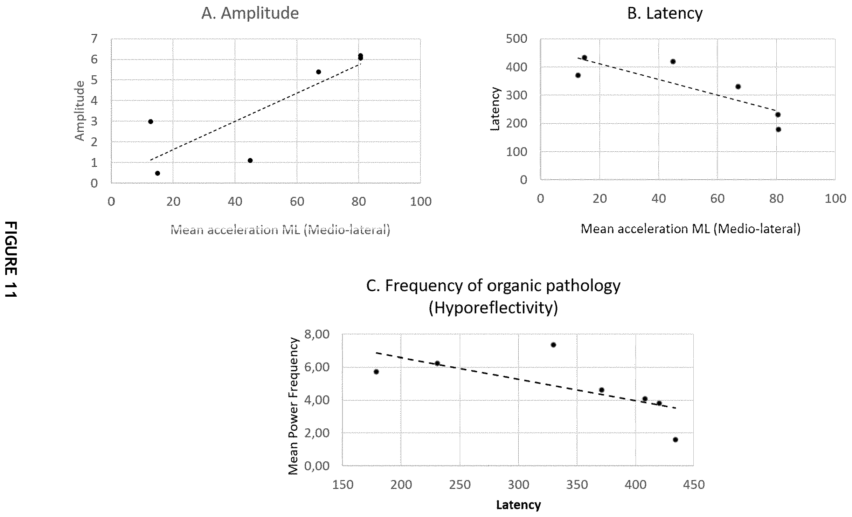

In hyporeflectivity patients, mediolateral body sway increased when the amplitude of convergence increases (see **Figure 11A****)** or when latency of convergence decreases (see **Figure 11B****).** Correlation was found between the latency of convergence and the mean power frequency, the longer the latency of the conversions the shorter the mean power frequency (see **Figure 11C****).**

### Discussion

The above results showed improvements particularly for divergence and more particularly in the standing position.

This is important as indeed the presence of vertigo in patients with their fear restricts somehow the capacity to move the eyes from near to far space which is done with the divergence movement before even undertaking any action.

The results show that transition from near to far space is done with shorter preparation time (shorter latency) and larger amplitude. The fact that such improvements are seen mostly at the standing position is also important because it indicates some part of multi-sensory facilitation of the binocular eye movement control by the vestibular and somatosensory signals coming from the ears and the body while in the standing position. Thus, these observations correlate with the subjective reports from several patients reporting better body self-control and more confidence while ambulating in the space in everyday life.

Further analysis of the postural and eye movement results shows that patients with functional origin of vertigo show smaller acceleration of ML body sway while doing vergence eye movements than while fixating. Such observations have been made previously in elderly with a falling history (Matheron E, Yang Q, Delpit-Baraut V, Dailly O, Kapoula Z. Active ocular vergence improves postural control in elderly as close viewing distance with or without a single cognitive task. Neurosci Lett. 2016 Jan 1;610:24-9) or even in patients with bilateral vestibular loss (Kapoula Z, Gaertner C, Yang Q, Denise P, Toupet M (2013) Vergence and Standing Balance in Subjects with Idiopathic Bilateral Loss of Vestibular Function. PLOS ONE 8(6): e66652).

Thus, many patients with functional origin of vertigo clearly can benefit from vergence eye movement rehabilitation as done with the REMOBI technology. The results from patients with organic origin confirm the link between eye movements and posture and indicate fragile link in these cases. Also, the mean power frequency is believed to be an indicator that less energy needed to keep posture stable (see Matheron E, Yang Q, Delpit-Baraut V, Dailly O, Kapoula Z. Active ocular vergence improves postural control in elderly as close viewing distance with or without a single cognitive task. Neurosci Lett. 2016 Jan 1;610:24-9). Presumably the patients capable to trigger their convergence with shorter latency, are also capable to control their postural stability by deploying less energy.

### Conclusions

All patients who experienced vertigo, regardless of their origin, consistently showed vergence disorders. This mediocre quality of vergence eye movements observed in the course of a vertiginous patient history is explained by the reciprocity highlighted between vestibular function and that of vergences. A specific rehabilitation of vergence movements (double step process) with the multi-sensory, audio-visual REMOBI device stimulating movements of the eyes in the real 3D space allows to recover the lost quality of these vergence movements, namely in terms of temporality, but also in some cases in terms of velocity and accuracy. This subjective patient reports highlight their improved self-confidence in ambulating in space during their everyday activity. Although the study is pilot, given the massive research evidence for a symbiosis between vergence and vestibular function, the vestibular reeducation devoted to the treatment of vertigo cannot therefore neglect the specific work of binocular eye movements. The REMOBI &AIDEAL technology invented is the only one enabling measure and rehabilitation of binocular vergence eye movements to targets in real 3D space without the known side effects of devices such as screens or virtual reality.

Theoretically, vergence disorders in these patients confirm the natural symbiosis that exists between vergence and vestibular function. Persistent abnormalities of vergence may contribute to the maintenance or recurrence of vertigo symptoms. The neuro-rehabilitation protocol with REMOBI (standing-seated) is integrative, stimulating both the binocular visuo-motor system, the cortical (frontal parietal) and subcortical network controlling this motor function, the musculoskeletal system as a whole, using stimuli in real space in three dimensions, as in everyday life. It allows the improvement of the temporality of the vergence and thus a better interaction between vestibular and postural functions.

### Example 2: Anomalies of vergence in patients with vertigo: diagnosis with REMOBI in 24 further patients

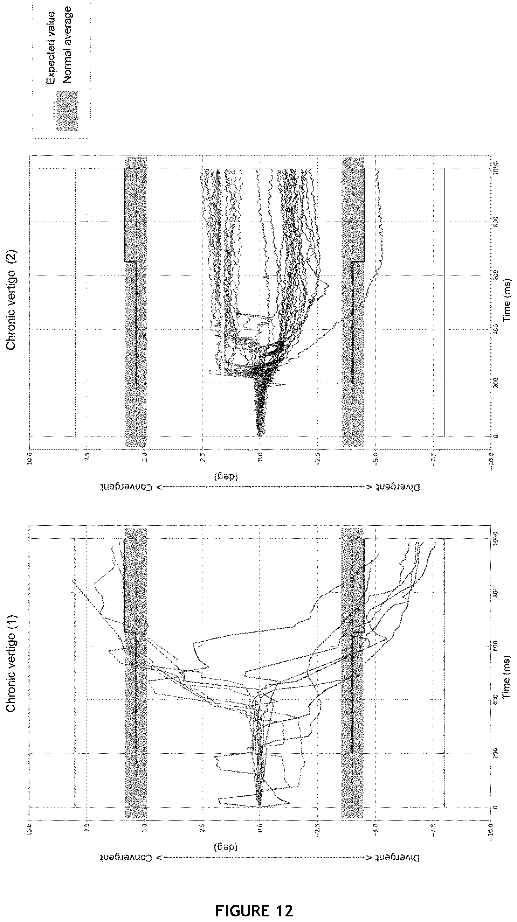

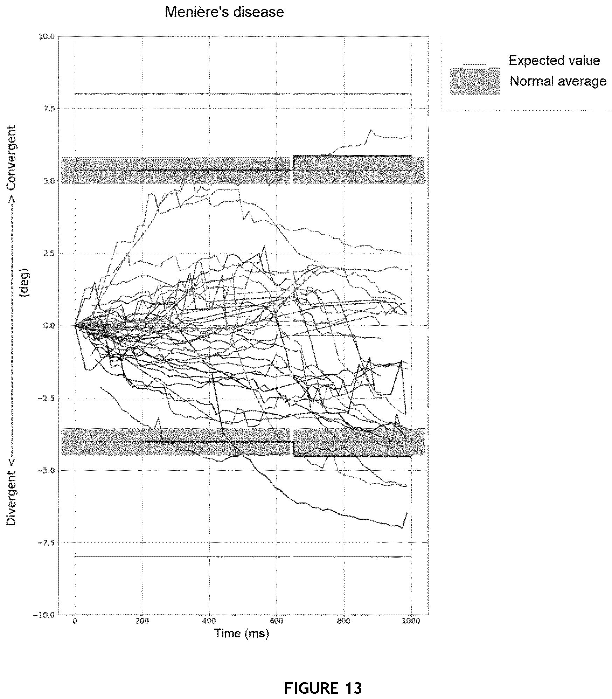

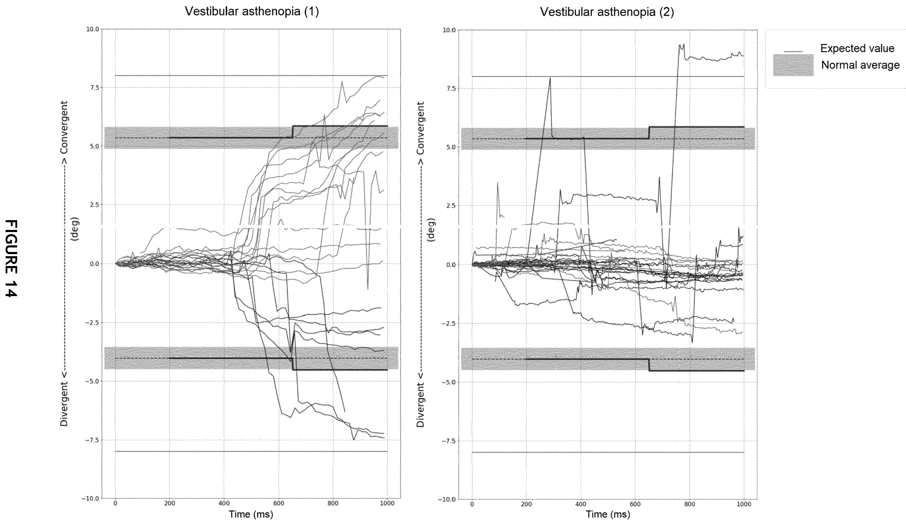

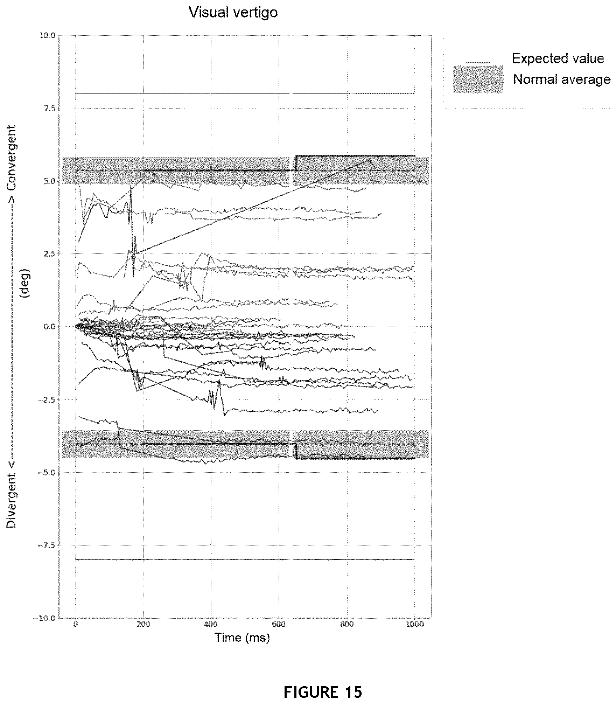

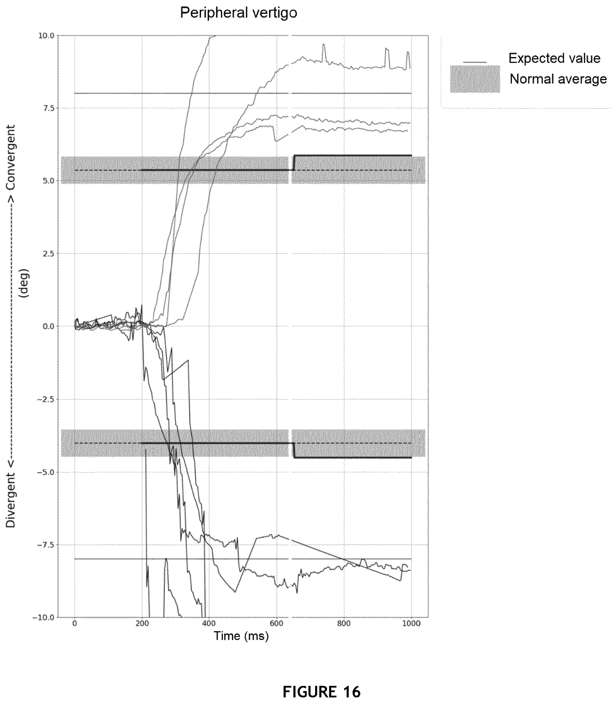

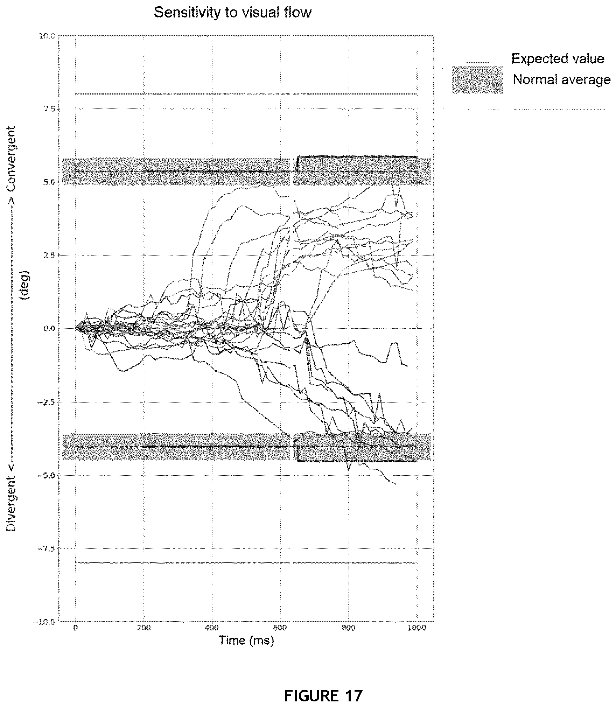

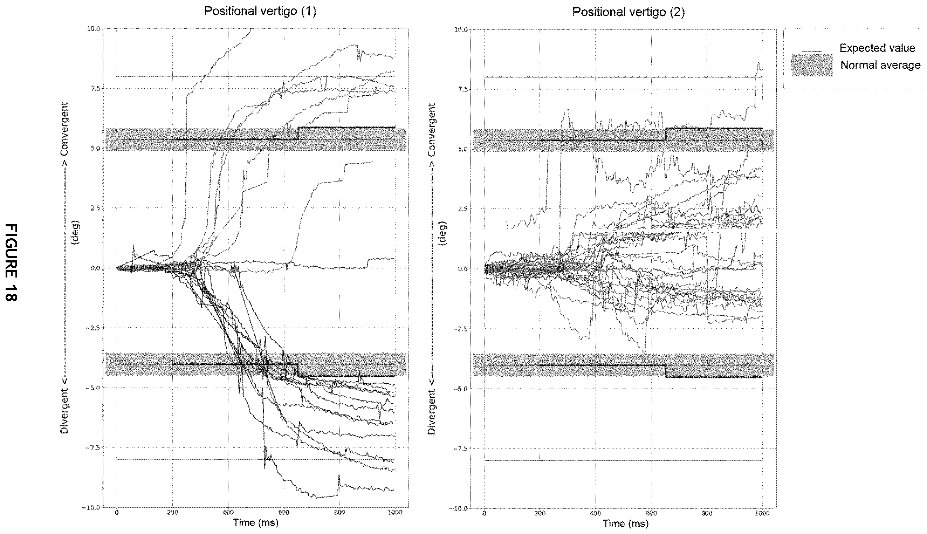

The vergences of 22 further individuals suffering from various types of vertigo were analyzed using the REMOBI device (vergence test is as disclosed in Example 1), including individuals suffering from:
- chronic vertigo (3 individuals, see exemplary abnormal vergence measurements in **Figure 12****),**
- chronic vertigo + Mesnière's disease (1 individual),
- chronic vertigo+ neuropsychologic disorders (1 individual),
- chronic vertigo+ sensitivity to visual flow (1 individual),
- Mesnière's disease (2 individuals, see exemplary abnormal vergence measurement in **Figure 13****),**
- vestibular asthenopia (5 individuals, see exemplary abnormal vergence measurements in **Figure 14****),**
- visual vertigo (2 individuals, see exemplary abnormal vergence measurement in **Figure 15****),**
- peripheral vertigo (1 individual, see exemplary abnormal vergence measurement in **Figure 16****),**
- sensitivity to visual flow (2 individuals, see exemplary abnormal vergence measurement in **Figure 17****),**
- positional vertigo (3 individuals see exemplary abnormal vergence measurements in **Figure 18****),** and
- vestibular asthenopia+ visual vertigo+ sensitivity to visual flow (1 individual).

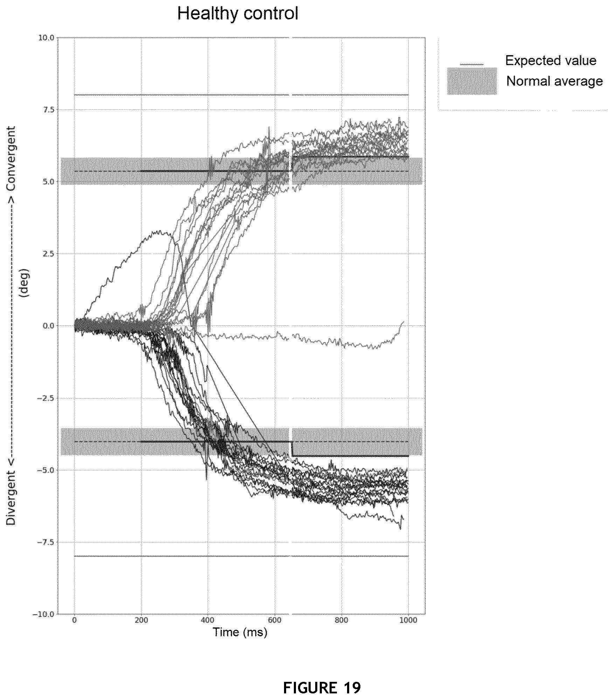

Trajectories of convergences and divergences from a healthy individual measured using REMOBI device are provided in **Figure 19** for comparison.

All of the 22 tested individuals suffering from the various types of vertigo showed abnormal vergences (very few adequate responses, high variability, either paucity of vergence or sometimes few exaggerated inappropriate responses, excepted 3 individuals suffering respectively from vestibular asthenopia, Mesniere's disease (moderate problem), and visual vertigo (moderate problem).

This confirms the importance of detecting vergence abnormalities in individuals suffering from vertigo with a device such as REMOBI, and also the interest of rehabilitation using the same device in vertigo individuals.

## Claims

1. A method for measuring binocular coordination and postural parameters in an individual suffering from vertigo, comprising:
a1) submitting the individual to ocular motor tests comprising two vergence tests, and optionally two saccade tests and/or two combined tests, one being performed with the individual in seated position and the other with the individual in standing position, and recording the eye movements of the left eye and the right eye of the individual during the two vergence tests, and optionally during the two saccade tests and/or two combined tests;
b1) submitting the individual to posture tests comprising:
i) the vergence test, and optionally the saccade test and/or the combined test, performed in standing position of step a1),
ii) a first fixation test at near, consisting in fixating a target for 20 to 40 seconds, preferably 30 seconds, at a first distance between 20 and 50 cm,
iii) a second fixation test at far, consisting in fixating a target for 20 to 40 seconds, preferably 30 seconds, at a second distance between 100 and 200 cm,
iv) a first vestibulo-ocular reflex (VOR) test at near, consisting in turning the head left and right while keeping fixating a target at a first distance between 20 and 50 cm, and
v) a second vestibulo-ocular reflex (VOR) test at far, consisting in turning the head left and right while keeping fixating a target at a second distance between 100 and 200 cm,
and recording the eye movements of the left eye and the right eye of the individual in posture tests i), iv) and v), recording body sway of the individual during the posture tests i) to iii); and recording head rotations during posture tests iv) and v);
c1) calculating, using a digital processing device, the value of one or more binocular coordination parameter from the records obtained in step a1) and the value of a one or more postural parameter from the records obtained in step b1);
wherein the two vergence tests are conducted using a binocular motor stimulation device configured to specifically and physically stimulate vergences and saccades, comprising visual or audiovisual targets placed in real space at eccentricities and depths of a surface;
wherein each of the two vergence tests comprises 30 to 50 trials of:
• visually and/or audiovisually stimulating the individual at a point located at the eyes level, in the central axis between the left eye and the right eye, at a first distance, and
• visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved.

2. The method according to claim 1, wherein the vergence test of step a1) comprises 30 to 50 trials of:
• visually and/or audiovisually stimulating the individual at a point located at the eyes level, in the central axis between the left eye and the right eye, at a distance of 40±5 cm for a period varying from 1400ms to 2000ms, and
• visually and/or audiovisually stimulating the individual at another point located at the eyes level, in the central axis between the left eye and the right eye, at a distance of 20±5 cm calling for a convergence movement or at a distance of 70 to 150 cm calling for a divergence movement during 1500 to 2500 ms, preferably 2000 ms, half of the trials calling for a convergence movement, the other half for a divergence movement, the trials calling for convergence and divergence movements being interleaved.

3. The method according to claim 1 or claim 2, wherein step a1) further comprises submitting the individual to two saccade tests, one being performed with the individual in seated position and the other with the individual in standing position, and recording the eye movements of the left eye and the right eye and body sway of the individual during the two saccade tests, wherein the two saccade tests are conducted using a binocular motor stimulation device configured to specifically and physically stimulate vergences and saccades, comprising visual or audiovisual targets placed in real space at eccentricities and depths of a surface, wherein each of the two saccade tests comprises 30 to 50 trials of:
• visually and/or audiovisually stimulating the individual at a point located at the eyes level, in the central axis between the left eye and the right eye, and
• visually and/or audiovisually stimulating the individual at another point located at the eyes level, on the left or on the right of the previous point, half of the trials being on the left, the other half on the right, the trials on the left and on the right being interleaved.

4. The method according to anyone of claims 1 to 3, wherein step a1) further comprises submitting the individual to two combined tests combining vergence and saccade movements, one being performed with the individual in seated position and the other with the individual in standing position, and recording the eye movements of the left eye and the right eye and body sway of the individual during the two combined tests, wherein the two combined tests are conducted using a binocular motor stimulation device configured to specifically and physically stimulate vergences and saccades, comprising visual or audiovisual targets placed in real space at eccentricities and depths of a surface,
wherein each of the two combined tests comprises 70 to 90 trials of:
• visually and/or audiovisually stimulating the individual at a point located at the eyes level, in the central axis between the left eye and the right eye, at a distance of 40 cm ± 5 cm (i.e. between 35 and 45 cm, preferably between 36 and 44 cm, between 37 and 43 cm, between 38 and 42 cm, between 39 and 41 cm, more preferably 40 cm) or at a distance of 150 cm ± 5 cm (i.e. between 35 and 45 cm, preferably between 36 and 44 cm, between 37 and 43 cm, between 38 and 42 cm, between 39 and 41 cm, more preferably 40 cm) for a period varying from 1400ms to 2000ms, and
• visually and/or audiovisually stimulating the individual during 1500 ms to 2000ms, preferably 2000 ms at another point located at the eyes level, either
∘ when starting from fixation point F1: at a distance of 150 cm ± 5 cm (i.e. between 35 and 45 cm, preferably between 36 and 44 cm, between 37 and 43 cm, between 38 and 42 cm, between 39 and 41 cm, more preferably 40 cm) and at 15° to 20°, preferably 20° of eccentricity on the left or on the right,
∘ when starting from fixation point F2: at a distance of 40 cm ± 5 cm (i.e. between 35 and 45 cm, preferably between 36 and 44 cm, between 37 and 43 cm, between 38 and 42 cm, between 39 and 41 cm, more preferably 40 cm) and at 15° to 20°, preferably 20° of eccentricity on the left or on the right,
wherein a quarter of the trials combine convergence and left saccade, a quarter of the trials combine convergence and right saccade, a quarter of the trials combine divergence and left saccade, and a quarter of the trials combine divergence and right saccade, the trials being (preferably randomly) interleaved.

5. The method according to anyone of claims 1 to 4, wherein the individual is submitted in step a1) to two vergence tests, two saccade tests, and two combined tests, one being performed with the individual in seated position and the other with the individual in standing position, all tests being conducted using a binocular motor stimulation device configured to specifically and physically stimulate vergences and saccades, comprising visual or audiovisual targets placed in real space at eccentricities and depths of a surface.

6. The method according to anyone of claims 1 to 5, wherein the vergence tests, and the saccade and combined tests, when present, are performed using audiovisual stimulation.

7. The method according to anyone of claims 1 to 6, wherein the binocular coordination parameter(s) calculated in step c1) comprise latency and total amplitude of convergences and/or divergences during the vergence tests, and optionally one or more other vergence parameter(s) during the vergence tests, one or more saccade parameter(s) during the saccade tests, one or more vergence parameter(s) during the combined tests, one or more saccade parameter(s) during the combined tests, and/or one or more binocular parameters during the first vestibulo-ocular reflex (VOR) test at near and the second VOR test at far, including one or more of the conjugate ((left eye+right eye)/2) amplitude of eye movements from peak to peak (left to right to left) and the disconjugate (left eye-right eye) amplitude of eye movements from peak to peak (left to right to left).

8. The method according to anyone of claims 1 to 7, wherein the postural parameters calculated in step c1) comprise one or more of the following body movement parameters:
• Body sway parameters for posture tests i) (vergence test in standing position), ii) and iii) (fixation tests at near and at far):
∘ Standardized area,
∘ Root-Mean-Square of the Medio-Lateral Body Sway,
∘ Root-Mean-Square of the Antero-Posterior Body Sway,
∘ Root-Mean-Square of the Medio-Lateral velocity,
∘ Root-Mean-Square of the Antero-Posterior velocity, and
∘ Mean power frequency ;
• Head rotation parameters for posture tests iv) and v) (VOR tests at near and far):
∘ average amplitude of head rotation (left/right, or up/down) and its variability,
∘ average gaze amplitude and its variability, and
∘ average gain of the VOR and its variability.

9. The method according to anyone of claims 1 to 6, wherein the binocular coordination parameters and postural parameters calculated in step c1) comprise:
• Vergence parameters during the two vergence tests, comprising latency of convergences and divergences and total amplitude of convergences and divergences, optionally further comprising one or more other vergence parameter(s) during the two vergence tests;
• Optionally, saccade parameters during the two saccade tests, such as one or more duration parameter, one or more amplitude parameter, and one or more velocity parameter;
• Optionally, vergence parameters during the two combined tests, such as one or more duration parameter, one or more amplitude parameter, and one or more velocity parameter;
• Optionally, saccade parameters during the two combined tests, such as one or more duration parameter, one or more amplitude parameter, and one or more velocity parameter;
• Optionally, one or more binocular parameters during the first vestibulo-ocular reflex (VOR) test at near and the second VOR test at far, including one or more of the conjugate ((left eye+right eye)/2) amplitude of eye movements from peak to peak (left to right to left) and the disconjugate (left eye-right eye) amplitude of eye movements from peak to peak (left to right to left), and
• Postural parameters during the posture tests, comprising one or more of:
∘ Body sway parameters for posture tests i) (vergence test in standing position), ii) and iii) (fixation tests at near and at far):
▪ Standardized area,
▪ Root-Mean-Square of the Medio-Lateral Body Sway,
▪ Root-Mean-Square of the Antero-Posterior Body Sway,
▪ Root-Mean-Square of the Medio-Lateral velocity,
▪ Root-Mean-Square of the Antero-Posterior velocity, and
▪ Mean power frequency ;
∘ Head rotation parameters for posture tests iv) and v) (VOR tests at near and far):
▪ average amplitude of head rotation (left/right, or up/down) and its variability,
▪ average gaze amplitude and its variability, and
▪ average gain of the VOR and its variability.

10. A method for detecting intrinsic ocular motor abnormalities and postural control abnormalities in an individual suffering from vertigo, comprising:
a2) providing previously obtained recordings of the eye movements of the left eye and the right eye of the individual during:
• ocular motor tests comprising two vergence tests, and optionally two saccade tests and/or two combined tests, one being performed with the individual in seated position and the other with the individual in standing position,
• posture tests comprising:
iv. a first vestibulo-ocular reflex (VOR) test at near, consisting in turning the head left and right while keeping fixating a target at a first distance between 20 and 50 cm, and
v. a second vestibulo-ocular reflex (VOR) test at far, consisting in turning the head left and right while keeping fixating a target at a second distance between 100 and 200 cm
b2) providing previously obtained recordings of body sway of the individual during posture tests comprising:
i) the vergence test, and optionally the saccade test and/or the combined test, performed in standing position of step a1),
ii) a first fixation test at near, consisting in fixating a target for 20 to 40 seconds, preferably 30 seconds, at a first distance between 20 and 50 cm,
iii) a second fixation test at far, consisting in fixating a target for 20 to 40 seconds, preferably 30 seconds, at a second distance between 100 and 200 cm,
iv) a first vestibulo-ocular reflex (VOR) test at near, consisting in turning the head left and right while keeping fixating a target at a first distance between 20 and 50 cm, and
v) a second vestibulo-ocular reflex (VOR) test at far, consisting in turning the head left and right while keeping fixating a target at a second distance between 100 and 200 cm,
c2) calculating, using a digital processing device, the value(s) of one or more binocular coordination parameter from the records provided in step a2) and the value(s) of one or more postural parameter(s) from the records provided in step b2);
d2) comparing the value(s) calculated in step c2) to one or more reference value(s) obtained in one or more individual(s) not suffering from vertigo;
e2) concluding to the presence of intrinsic ocular motor abnormalities in the individual suffering from vertigo if one or more binocular coordination parameter calculated in step c2) is significantly different from the one or more reference value(s) obtained in one or more individual(s) not suffering from vertigo; and
f2) concluding to the presence of postural control abnormalities in the individual suffering from vertigo if one or more postural parameter(s) calculated in step c2) is significantly different from the one or more reference value(s) obtained in one or more individual(s) not suffering from vertigo;
wherein the two vergence tests from which the recordings are provided in step a2) have been conducted using a binocular motor stimulation device configured to specifically and physically stimulate vergences and saccades, comprising visual or audiovisual targets placed in real space at eccentricities and depths of a surface;
wherein each of the two vergence tests which recordings are provided comprised 30 to 50 trials of:
• visually and/or audiovisually stimulating the individual at a point located at the eyes level, in the central axis between the left eye and the right eye, at a first distance, and
• visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved.

11. The method according to claim 10, wherein the recordings provided in step a2) have been obtained as in step a1) or b1) of the method according to any one of claims 1 to 9 and the recordings provided in step b2) have been obtained as in step b1) of the method according to any one of claims 1 to 9.

12. The method according to any one of claims 1 to 11, wherein the individual suffering from vertigo is suffering from vertigo of organic origin.

13. The method according to any one of claims 1 to 11, wherein the individual suffering from vertigo is suffering from vertigo of functional origin.

14. The method according to claim 13, wherein the individual suffering from vertigo is suffering from vestibular asthenopia or Meniere's disease.

## Patentansprüche

1. Verfahren zum Messen der binokularen Koordination und der Haltungsparameter bei einer Person, die unter Schwindel leidet, umfassend:
a1) Durchführen eines Augenmotoriktests an der Person, der zwei Vergenztests und optional zwei Sakkadentests und/oder zwei kombinierte Tests umfassen, wobei einer mit der Person in sitzender Position und der andere mit der Person in stehender Position durchgeführt wird, und Aufzeichnen der Augenbewegungen des linken Auges und des rechten Auges der Person während der beiden Vergenztests und optional während der beiden Sakkadentests und/oder der beiden kombinierten Tests;
b1) Durchführen von Haltungstests an der Person, die Folgendes umfassen:
i) den Vergenztest und optional den Sakkadentest und/oder den kombinierten Test, die in stehender Position nach Schritt a1) durchgeführt werden,
ii) einen ersten Fixierungstest in dem Nahbereich, bei dem ein Ziel für 20 bis 40 Sekunden, vorzugsweise 30 Sekunden, in einem ersten Abstand zwischen 20 und 50 cm fixiert wird,
iii) einen zweiten Fixierungstest in dem Fernbereich, bei dem ein Ziel für 20 bis 40 Sekunden, vorzugsweise 30 Sekunden, in einem Abstand von 100 bis 200 cm fixiert wird,
iv) einen ersten vestibulookulären Reflextest (VOR-Test) in dem Nahbereich, bei dem der Kopf nach links und rechts gedreht wird, während ein Ziel in einem ersten Abstand zwischen 20 und 50 cm fixiert wird, und
v) einen zweiten vestibulo-okulären Reflextest (VOR-Test) in dem Fernbereich, bei dem der Kopf nach links und rechts gedreht wird und gleichzeitig ein Ziel in einem zweiten Abstand zwischen 100 und 200 cm fixiert wird,
und Aufzeichnen der Augenbewegungen des linken Auges und des rechten Auges der Person bei den Haltungstests i), iv) und v), Aufzeichnen der Körperschwankung der Person während der Haltungstests i) bis iii); und Aufzeichnen der Kopfrotationen während der Haltungstests iv) und v);
c1) unter Verwendung einer digitalen Verarbeitungsvorrichtung, Berechnen des Werts eines oder mehrerer binokularer Koordinationsparameter aus den in Schritt a1) erhaltenen Aufzeichnungen und des Werts eines oder mehrerer Haltungsparameter aus den in Schritt b1) erhaltenen Aufzeichnungen;
wobei die beiden Vergenztests unter Verwendung einer binokularen motorischen Stimulationsvorrichtung durchgeführt werden, die so konfiguriert ist, dass sie Vergenzen und Sakkaden spezifisch und physikalisch stimuliert, und die visuelle oder audiovisuelle Ziele umfasst, die in dem realen Raum an Exzentrizitäten und Tiefen einer Oberfläche platziert sind;
wobei jeder der beiden Vergenztests aus 30 bis 50 Versuchen aus Folgendem besteht:
• visuellem und/oder audiovisuellem Stimulieren der Person an einem Punkt auf Augenhöhe, in der Mittelachse zwischen dem linken und dem rechten Auge, in einem ersten Abstand,
und
• visuellem und/oder audiovisuellem Stimulieren der Person an einem anderen Punkt auf Augenhöhe, in der Mittelachse zwischen dem linken und dem rechten Auge, in einem anderen Abstand, der eine Konvergenz- oder Divergenzbewegung erfordert, wobei die Hälfte der Versuche eine Konvergenzbewegung und die andere Hälfte eine Divergenzbewegung erfordert, wobei die Versuche, die Konvergenz- und Divergenzbewegungen erfordern, miteinander abgewechselt werden.

2. Verfahren nach Anspruch 1, wobei der Vergenztest in Schritt a1) aus 30 bis 50 Versuchen aus Folgendem besteht:
• visuellem und/oder audiovisuellem Stimulieren der Person an einem Punkt auf Augenhöhe, in der Mittelachse zwischen dem linken und dem rechten Auge, in einem Abstand von 40 ± 5 cm für einen Zeitraum von 1400 ms bis 2000 ms, und
• visuellem und/oder audiovisuellem Stimulieren der Person an einem anderen Punkt auf Augenhöhe, in der Mittelachse zwischen dem linken und dem rechten Auge, in einem Abstand von 20 ± 5 cm, der eine Konvergenzbewegung erfordert, oder in einem Abstand von 70 bis 150 cm, der eine Divergenzbewegung erfordert, über einen Zeitraum von 1500 bis 2500 ms, vorzugsweise 2000 ms, wobei die Hälfte der Versuche eine Konvergenzbewegung und die andere Hälfte eine Divergenzbewegung erfordert, wobei die Versuche, die Konvergenz- und Divergenzbewegungen erfordern, miteinander abgewechselt werden.

3. Verfahren nach Anspruch 1 oder Anspruch 2, wobei Schritt a1) ferner das Durchführen zweier Sakkadentests an der Person, wobei einer mit der Person in sitzender Position und der andere mit der Person in stehender Position durchgeführt wird, und das Aufzeichnen der Augenbewegungen des linken Auges und des rechten Auges sowie der Körperschwankung der Person während der beiden Sakkadentests umfasst, wobei die beiden Sakkadentests unter Verwendung einer binokularen motorischen Stimulationsvorrichtung durchgeführt werden, die so konfiguriert ist, dass sie Vergenzen und Sakkaden spezifisch und physikalisch stimuliert, und die visuelle oder audiovisuelle Ziele umfasst, die im realen Raum an Exzentrizitäten und Tiefen einer Oberfläche platziert sind, wobei jeder der beiden Sakkadentests aus 30 bis 50 Versuchen aus Folgendem besteht:
• visuellem und/oder audiovisuellem Stimulieren der Person an einem Punkt auf Augenhöhe in der Mittelachse zwischen dem linken und dem rechten Auge und
• visuellem und/oder audiovisuellem Stimulieren der Person an einem anderen Punkt auf Augenhöhe, links oder rechts von dem vorherigen Punkt, wobei die Hälfte der Versuche links, die andere Hälfte rechts und die Versuche links und rechts eingebunden sind.

4. Verfahren nach einem der Ansprüche 1 bis 3, wobei Schritt a1) ferner das Durchführen zweier kombinierter Tests an der Person, bei denen Vergenz- und Sakkadenbewegungen kombiniert werden, wobei einer mit der Person in sitzender Position und der andere mit der Person in stehender Position durchgeführt wird, und das Aufzeichnen der Augenbewegungen des linken Auges und des rechten Auges und der Körperschwankung der Person während der beiden kombinierten Tests umfasst, wobei die beiden kombinierten Tests unter Verwendung einer binokularen motorischen Stimulationsvorrichtung durchgeführt werden, die so konfiguriert ist, dass sie Vergenzen und Sakkaden spezifisch und physikalisch stimuliert, und die visuelle oder audiovisuelle Ziele umfasst, die in dem realen Raum an Exzentrizitäten und Tiefen einer Oberfläche platziert sind, wobei jeder der beiden kombinierten Tests aus 70 bis 90 Versuchen aus Folgendem besteht:
• visuellem und/oder audiovisuellem Stimulieren der Person an einem Punkt auf Augenhöhe in der Mittelachse zwischen dem linken Auge und dem rechten Auge in einem Abstand von 40 cm ± 5 cm (d. h. zwischen 35 und 45 cm, vorzugsweise zwischen 36 und 44 cm, zwischen 37 und 43 cm, zwischen 38 und 42 cm, zwischen 39 und 41 cm, bevorzugter 40 cm) oder in einem Abstand von 150 cm ± 5 cm (d. h. zwischen 35 und 45 cm, vorzugsweise zwischen 36 und 44 cm, zwischen 37 und 43 cm, zwischen 38 und 42 cm, zwischen 39 und 41 cm, bevorzugter 40 cm) für einen Zeitraum von 1400 ms bis 2000 ms, und
• visuellem und/oder audiovisuellem Stimulieren der Person für 1500 ms bis 2000 ms, vorzugsweise 2000 ms, an einem anderen Punkt auf Augenhöhe, entweder
o ausgehend von dem Fixierungspunkt F1: in einem Abstand von 150 cm ± 5 cm (d. h. zwischen 35 und 45 cm, vorzugsweise zwischen 36 und 44 cm, zwischen 37 und 43 cm, zwischen 38 und 42 cm, zwischen 39 und 41 cm, bevorzugter 40 cm) und bei einer Exzentrizität von 15° bis 20°, vorzugsweise 20°, links oder rechts,
o ausgehend von dem Fixierungspunkt F2: in einem Abstand von 40 cm ± 5 cm (d. h. zwischen 35 und 45 cm, vorzugsweise zwischen 36 und 44 cm, zwischen 37 und 43 cm, zwischen 38 und 42 cm, zwischen 39 und 41 cm, bevorzugter 40 cm) und bei einer Exzentrizität von 15° bis 20°, vorzugsweise 20°, links oder rechts,
wobei ein Viertel der Versuche Konvergenz mit einer linken Sakkade kombiniert, ein Viertel der Versuche Konvergenz mit einer rechten Sakkade kombiniert, ein Viertel der Versuche Divergenz mit einer linken Sakkade kombiniert und ein Viertel der Versuche Divergenz mit einer rechten Sakkade kombiniert, wobei die Versuche (vorzugsweise zufällig) miteinander abgewechselt werden.

5. Verfahren nach einem der Ansprüche 1 bis 4, wobei in Schritt a1) zwei Vergenztests, zwei Sakkadentests und zwei kombinierte Tests an der Person durchgeführt werden, wobei einer mit der Person in sitzender Position und der andere mit der Person in stehender Position durchgeführt wird, wobei alle Tests unter Verwendung einer binokularen motorischen Stimulationsvorrichtung durchgeführt werden, die so konfiguriert ist, dass sie Vergenzen und Sakkaden spezifisch und physikalisch stimuliert, und die visuelle oder audiovisuelle Ziele umfasst, die in dem realen Raum an Exzentrizitäten und Tiefen einer Oberfläche platziert werden.

6. Verfahren nach einem der Ansprüche 1 bis 5, wobei die Vergenztests sowie die Sakkaden- und kombinierten Tests, sofern vorhanden, unter Verwendung audiovisueller Stimulation durchgeführt werden.

7. Verfahren nach einem der Ansprüche 1 bis 6, wobei der beziehungsweise die in Schritt c1) berechneten binokularen Koordinationsparameter die Latenz und die Gesamtamplitude von Konvergenzen und/oder Divergenzen während der Vergenztests sowie optional einen oder mehrere andere Vergenzparameter während der Vergenztests, einen oder mehrere Sakkadenparameter während der Sakkadentests, einen oder mehrere Vergenzparameter während der kombinierten Tests, einen oder mehrere Sakkadenparameter während der kombinierten Tests und/oder einen oder mehrere binokulare Parameter während des ersten vestibulookulären Reflextests (VOR-Tests) im Nahbereich und des zweiten VOR-Tests im Fernbereich umfassen, einschließlich einer oder mehrerer der konjugierten ((linkes Auge + rechtes Auge)/2) Amplituden der Augenbewegungen von Spitze zu Spitze (von links nach rechts nach links) und der diskonjugierten (linkes Auge - rechtes Auge) Amplitude der Augenbewegungen von Spitze zu Spitze (von links nach rechts nach links).

8. Verfahren nach einem der Ansprüche 1 bis 7, wobei die in Schritt c1) berechneten Haltungsparameter einen oder mehrere der folgenden Körperbewegungsparameter umfassen:
• Körperschwankungsparameter für Haltungstests i) (Vergenztest in stehender Position), ii) und iii) (Fixierungstests in dem Nah- und Fernbereich):
∘ standardisierter Bereich,
∘ quadratischer Mittelwert der mediolateralen Körperschwankung,
∘ quadratischer Mittelwert der anteroposterioren Körperschwankung,
∘ quadratischer Mittelwert der mediolateralen Geschwindigkeit,
∘ quadratischer Mittelwert der anteroposterioren Geschwindigkeit, und
∘ mittlere Netzfrequenz;
• Kopfrotationsparameter für Haltungstests iv) und v) (VOR-Tests in dem Nah- und Fernbereich):
∘ mittlere Amplitude der Kopfrotation (links/rechts oder auf/ab) und ihre Variabilität,
∘ durchschnittliche Blickamplitude und ihre Variabilität, und
∘ durchschnittliche Verstärkung des VOR und dessen Variabilität.

9. Verfahren nach einem der Ansprüche 1 bis 6, wobei die in Schritt c1) berechneten binokularen Koordinationsparameter und Haltungsparameter Folgendes umfassen:
• Vergenzparameter während der beiden Vergenztests, bestehend aus der Latenz von Konvergenzen und Divergenzen sowie der Gesamtamplitude von Konvergenzen und Divergenzen, optional zusätzlich bestehend aus einem oder mehreren weiteren Vergenzparametern während der beiden Vergenztests;
• optional Sakkadenparameter während der beiden Sakkadentests, wie ein oder mehrere Dauerparameter, ein oder mehrere Amplitudenparameter und ein oder mehrere Geschwindigkeitsparameter;
• optional Vergenzparameter während der beiden kombinierten Tests, wie ein oder mehrere Dauerparameter, ein oder mehrere Amplitudenparameter und ein oder mehrere Geschwindigkeitsparameter;
• optional Sakkadenparameter während der beiden kombinierten Tests, wie ein oder mehrere Dauerparameter, ein oder mehrere Amplitudenparameter und ein oder mehrere Geschwindigkeitsparameter;
• optional einen oder mehrere binokulare Parameter während des ersten vestibulookulären Reflextests (VOR-Tests) in dem Nahbereich und des zweiten VOR-Tests in dem Fernbereich, einschließlich einer oder mehrerer der konjugierten ((linkes Auge + rechtes Auge) /2) Amplituden der Augenbewegungen von Spitze zu Spitze (von links nach rechts nach links) und der diskonjugierten (linkes Auge - rechtes Auge) Amplituden der Augenbewegungen von Spitze zu Spitze (von links nach rechts nach links), und
• Haltungsparameter während der Haltungstests, bestehend aus einem oder mehreren von Folgendem:
o Körperschwankungsparameter für Haltungstests i) (Vergenztest in stehender Position), ii) und iii) (Fixierungstests in dem Nah- und Fernbereich):
▪ standardisierter Bereich,
▪ quadratischer Mittelwert der mediolateralen Körperschwankung,
▪ quadratischer Mittelwert der anteroposterioren Körperschwankung,
▪ quadratischer Mittelwert der mediolateralen Geschwindigkeit,
▪ quadratischer Mittelwert der anteroposterioren Geschwindigkeit, und
▪ mittlere Netzfrequenz;
o Kopfrotationsparameter für Haltungstests iv) und v) (VOR-Tests in dem Nah- und Fernbereich):
▪ mittlere Amplitude der Kopfrotation (links/rechts oder auf/ab) und ihre Variabilität,
▪ durchschnittliche Blickamplitude und ihre Variabilität, und
▪ durchschnittliche Verstärkung des VOR und dessen Variabilität.

10. Verfahren zum Erkennen von intrinsischen okulomotorischen Anomalien und Anomalien der Haltungssteuerung bei einer Person, die unter Schwindel leidet, umfassend:
a2) Bereitstellen von zuvor erhaltenen Aufzeichnungen der Augenbewegungen des linken Auges und rechten Auges der Person während:
• Augenmotoriktests, die zwei Vergenztests und optional zwei Sakkadentests und/oder zwei kombinierte Tests umfassen, wobei einer mit der Person in sitzender Position und der andere mit der Person in stehender Position durchgeführt wird,
• Haltungstests, umfassend:
iv. einen ersten vestibulookulären Reflextest (VOR-Test) in dem Nahbereich, bei dem der Kopf nach links und rechts gedreht wird, während ein Ziel in einem ersten Abstand zwischen 20 und 50 cm fixiert wird, und
v. einen zweiten vestibulookulären Reflextest (VOR-Test) in dem Fernbereich, bei dem der Kopf nach links und rechts gedreht wird und gleichzeitig ein Ziel in einem zweiten Abstand zwischen 100 und 200 cm fixiert wird,
b2) Bereitstellen von zuvor erhaltenen Aufzeichnungen der Körperschwankung der Person während der Haltungstests, die Folgendes umfassen:
i) den Vergenztest und optional den Sakkadentest und/oder den kombinierten Test, die in stehender Position nach Schritt a1) durchgeführt werden,
ii) einen ersten Fixierungstest in dem Nahbereich, bei dem ein Ziel für 20 bis 40 Sekunden, vorzugsweise 30 Sekunden, in einem ersten Abstand zwischen 20 und 50 cm fixiert wird,
iii) einen zweiten Fixierungstest in dem Fernbereich, bei dem ein Ziel für 20 bis 40 Sekunden, vorzugsweise 30 Sekunden, in einem Abstand von 100 bis 200 cm fixiert wird,
iv) einen ersten vestibulookulären Reflextest (VOR-Test) in dem Nahbereich, bei dem der Kopf nach links und rechts gedreht wird, während ein Ziel in einem ersten Abstand zwischen 20 und 50 cm fixiert wird, und
v) einen zweiten vestibulo-okulären Reflextest (VOR-Test) in dem Fernbereich, bei dem der Kopf nach links und rechts gedreht wird und gleichzeitig ein Ziel in einem zweiten Abstand zwischen 100 und 200 cm fixiert wird,
c2) Berechnen, unter Verwendung einer digitalen Verarbeitungsvorrichtung, des beziehungsweise der Werte eines oder mehrerer binokularer Koordinationsparameter aus den in Schritt a2) bereitgestellten Aufzeichnungen sowie des beziehungsweise der Werte eines oder mehrerer Haltungsparameter aus den in Schritt b2) bereitgestellten Aufzeichnungen;
c2) Vergleichen des beziehungsweise der in Schritt b2) berechneten Werte mit einem oder mehreren Referenzwerten, die bei einer oder mehreren Personen ermittelt wurden, die nicht unter Schwindel leiden; und
e2) Feststellen des Vorhandenseins von intrinsischen okulomotorischen Anomalien bei der Person, die unter Schwindel leidet, wenn ein oder mehrere in Schritt c2) berechnete binokulare Koordinationsparameter signifikant von dem einen oder den mehreren Referenzwerten abweichen, die bei einer oder mehreren Personen, die nicht unter Schwindel leiden, erhalten wurden; und
f2) Feststellen des Vorhandenseins von Anomalien der Körperhaltung bei der Person, die unter Schwindel leidet, wenn ein oder mehrere in Schritt c2) berechnete Haltungsparameter signifikant von dem einen oder den mehreren Referenzwerten abweichen, die bei einer oder mehreren Personen, die nicht unter Schwindel leiden, erhalten wurden;
wobei die beiden Vergenztests, aus denen die Aufzeichnungen in Schritt a2) bereitgestellt werden, unter Verwendung einer binokularen motorischen Stimulationsvorrichtung durchgeführt wurden, die so konfiguriert ist, dass sie Vergenzen und Sakkaden spezifisch und physikalisch stimuliert, und die visuelle oder audiovisuelle Ziele umfasst, die in dem realen Raum an Exzentrizitäten und Tiefen einer Oberfläche platziert sind;
wobei jeder der beiden Vergenztests, für die Aufzeichnungen bereitgestellt werden, aus 30 bis 50 Versuchen aus Folgendem besteht:
• visuellem und/oder audiovisuellem Stimulieren der Person an einem Punkt auf Augenhöhe, in der Mittelachse zwischen dem linken und dem rechten Auge, in einem ersten Abstand, und
• visuellem und/oder audiovisuellem Stimulieren der Person an einem anderen Punkt auf Augenhöhe, in der Mittelachse zwischen dem linken und dem rechten Auge, in einem anderen Abstand, der eine Konvergenz- oder Divergenzbewegung erfordert, wobei die Hälfte der Versuche eine Konvergenzbewegung und die andere Hälfte eine Divergenzbewegung erfordert, wobei die Versuche, die Konvergenz- und Divergenzbewegungen erfordern, miteinander abgewechselt werden.

11. Verfahren nach Anspruch 10, wobei die in Schritt a2) bereitgestellten Aufzeichnungen wie in Schritt a1) oder b1) des Verfahrens nach einem der Ansprüche 1 bis 9 erhalten wurden und die in Schritt b2) bereitgestellten Aufzeichnungen wie in Schritt b1) des Verfahrens nach einem der Ansprüche 1 bis 9 erhalten wurden.

12. Verfahren nach einem der Ansprüche 1 bis 11, wobei die unter Schwindel leidende Person unter Schwindel organischen Ursprungs leidet.

13. Verfahren nach einem der Ansprüche 1 bis 11, wobei die unter Schwindel leidende Person unter Schwindel funktionellen Ursprungs leidet.

14. Verfahren nach Anspruch 13, wobei die unter Schwindel leidende Person an vestibulärer Asthenopie oder Morbus Menière leidet.

## Revendications

1. Procédé pour mesurer des paramètres posturaux et de coordination binoculaire chez un individu souffrant de vertige, comprenant :
a1) la soumission de l'individu à des tests oculomoteurs comprenant deux tests de vergence, et facultativement deux tests de saccade et/ou deux tests combinés, l'un étant réalisé avec l'individu en position assise et l'autre avec l'individu en position debout, et l'enregistrement des mouvements oculaires de l'œil gauche et de l'œil droit de l'individu lors des deux tests de vergence, et facultativement lors des deux tests de saccade et/ou des deux tests combinés ;
b1) la soumission de l'individu à des tests posturaux comprenant :
i) le test de vergence, et facultativement le test de saccade et/ou le test combiné, réalisé en position debout à l'étape a1),
ii) un premier test de fixation de près, consistant à fixer une cible pendant 20 à 40 secondes, de préférence 30 secondes, à une distance initiale comprise entre 20 et 50 cm,
iii) un deuxième test de fixation de loin, consistant à fixer une cible pendant 20 à 40 secondes, de préférence 30 secondes, à une deuxième distance comprise entre 100 et 200 cm,
iv) un premier test du réflexe vestibulo-oculaire (RVO) de près, consistant à tourner la tête à gauche et à droite tout en fixant une cible à une première distance comprise entre 20 et 50 cm, et
v) un deuxième test du réflexe vestibulo-oculaire (RVO) de loin, consistant à tourner la tête à gauche et à droite tout en fixant une cible à une deuxième distance comprise entre 100 et 200 cm,
et l'enregistrement des mouvements oculaires de l'œil gauche et de l'œil droit de l'individu lors des tests posturaux i), iv) et v), l'enregistrement du balancement du corps de l'individu lors des tests posturaux i) à iii) ; et l'enregistrement de rotations de la tête lors des tests posturaux iv) et v) ;
c1) le calcul, à l'aide d'un dispositif de traitement numérique, de la valeur d'un ou plusieurs paramètres de coordination binoculaire à partir des enregistrements obtenus à l'étape a1) et de la valeur d'un ou plusieurs paramètres posturaux à partir des enregistrements obtenus à l'étape b1) ;
dans lequel les deux tests de vergence sont effectués à l'aide d'un dispositif de stimulation motrice binoculaire configuré pour stimuler spécifiquement et physiquement les vergences et les saccades, comprenant des cibles visuelles ou audiovisuelles placées dans l'espace réel à des excentricités et des profondeurs d'une surface ;
dans lequel chacun des deux tests de vergence comprend 30 à 50 essais de :
• stimulation visuelle et/ou audiovisuelle de l'individu à un point situé au niveau des yeux, dans l'axe central entre l'œil gauche et l'œil droit, à une première distance, et
• stimulation visuelle et/ou audiovisuelle de l'individu à un autre point situé au niveau des yeux, dans l'axe central entre l'œil gauche et l'œil droit, à une distance différente nécessitant un mouvement de convergence ou un mouvement de divergence, la moitié des essais nécessitant un mouvement de convergence, l'autre moitié un mouvement de divergence, les essais nécessitant des mouvements de convergence et de divergence étant entrelacés.

2. Procédé selon la revendication 1, dans lequel le test de vergence de l'étape a1) comprend 30 à 50 essais de :
• stimulation visuelle et/ou audiovisuelle de l'individu à un point situé au niveau des yeux, dans l'axe central entre l'œil gauche et l'œil droit, à une distance de 40±5 cm pendant une période variant de 1 400 ms à 2 000 ms, et
• stimulation visuelle et/ou audiovisuelle de l'individu à un autre point situé au niveau des yeux, dans l'axe central entre l'œil gauche et l'œil droit, à une distance de 20±5 cm nécessitant un mouvement de convergence, ou à une distance de 70 à 150 cm nécessitant un mouvement de divergence, pendant 1 500 à 2 500 ms, de préférence 2 000 ms, la moitié des essais nécessitant un mouvement de convergence, l'autre moitié un mouvement de divergence, les essais nécessitant des mouvements de convergence et de divergence étant entrelacés.

3. Procédé selon la revendication 1 ou la revendication 2, dans lequel l'étape a1) comprend en outre la soumission de l'individu à deux tests de saccade, l'un étant réalisé avec l'individu en position assise et l'autre avec l'individu en position debout, et l'enregistrement des mouvements oculaires de l'œil gauche et de l'œil droit et du balancement du corps de l'individu lors des deux tests de saccade, dans lequel les deux tests de saccade sont effectués à l'aide d'un dispositif de stimulation motrice binoculaire configuré pour stimuler spécifiquement et physiquement les vergences et les saccades, comprenant des cibles visuelles ou audiovisuelles placées dans l'espace réel à des excentricités et des profondeurs d'une surface, dans lequel chacun des deux tests de saccade comprend 30 à 50 essais de :
• stimulation visuelle et/ou audiovisuelle de l'individu à un point situé au niveau des yeux, dans l'axe central entre l'œil gauche et l'œil droit, et
• stimulation visuelle et/ou audiovisuelle de l'individu à un autre point situé au niveau des yeux, à gauche ou à droite du point précédent, la moitié des essais étant effectués à gauche et l'autre moitié à droite, les essais à gauche et à droite étant entrelacés.

4. Procédé selon l'une quelconque des revendications 1 à 3, dans lequel l'étape a1) comprend en outre la soumission de l'individu à deux tests combinés combinant des mouvements de vergence et de saccade, l'un étant réalisé avec l'individu en position assise et l'autre avec l'individu en position debout, et l'enregistrement des mouvements oculaires de l'œil gauche et de l'œil droit et du balancement du corps de l'individu lors des deux tests combinés, dans lequel les deux tests combinés sont effectués à l'aide d'un dispositif de stimulation motrice binoculaire configuré pour stimuler spécifiquement et physiquement les vergences et les saccades, comprenant des cibles visuelles ou audiovisuelles placées dans l'espace réel à des excentricités et des profondeurs d'une surface, dans lequel chacun des deux tests combinés comprend 70 à 90 essais de :
• stimulation visuelle et/ou audiovisuelle de l'individu à un point situé au niveau des yeux, dans l'axe central entre l'œil gauche et l'œil droit, à une distance de 40 cm ± 5 cm (soit entre 35 et 45 cm, de préférence entre 36 et 44 cm, entre 37 et 43 cm, entre 38 et 42 cm, entre 39 et 41 cm, plus préférentiellement 40 cm) ou à une distance de 150 cm ± 5 cm (soit entre 35 et 45 cm, de préférence entre 36 et 44 cm, entre 37 et 43 cm, entre 38 et 42 cm, entre 39 et 41 cm, plus préférentiellement 40 cm) pendant une période variant de 1400 ms à 2000 ms, et
• stimulation visuelle et/ou audiovisuelle de l'individu pendant 1 500 ms à 2 000 ms, de préférence 2 000 ms à un autre point situé au niveau des yeux, soit
o en partant du point de fixation F1 : à une distance de 150 cm ± 5 cm (soit entre 35 et 45 cm, de préférence entre 36 et 44 cm, entre 37 et 43 cm, entre 38 et 42 cm, entre 39 et 41 cm, plus préférentiellement 40 cm) et à une excentricité de 15° à 20°, de préférence 20° à gauche ou à droite,
o en partant du point de fixation F2 : à une distance de 40 cm ± 5 cm (soit entre 35 et 45 cm, de préférence entre 36 et 44 cm, entre 37 et 43 cm, entre 38 et 42 cm, entre 39 et 41 cm, plus préférentiellement 40 cm) et à une excentricité de 15° à 20°, de préférence 20° à gauche ou à droite,
dans lequel un quart des essais combinent convergence et saccade vers la gauche, un quart des essais combinent convergence et saccade vers la droite, un quart des essais combinent divergence et saccade vers la gauche, et un quart des essais combinent divergence et saccade vers la droite, les essais étant (de préférence aléatoirement) entrelacés.

5. Procédé selon l'une quelconque des revendications 1 à 4, dans lequel l'individu est soumis, à l'étape a1), à deux tests de vergence, à deux tests de saccade et à deux tests combinés, l'un étant réalisé avec l'individu en position assise et l'autre avec l'individu en position debout, tous les tests étant effectués à l'aide d'un dispositif de stimulation motrice binoculaire configuré pour stimuler spécifiquement et physiquement les vergences et les saccades, comprenant des cibles visuelles ou audiovisuelles placées dans l'espace réel à des excentricités et des profondeurs d'une surface.

6. Procédé selon l'une quelconque des revendications 1 à 5, dans lequel les tests de vergence, et les tests de saccade et les tests combinés, le cas échéant, sont réalisés à l'aide d'une stimulation audiovisuelle.

7. Procédé selon l'une quelconque des revendications 1 à 6, dans lequel le ou les paramètres de coordination binoculaire calculés à l'étape c1) comprennent la latence et l'amplitude totale de convergences et/ou de divergences lors des tests de vergence, et facultativement un ou plusieurs autres paramètres de vergence lors des tests de vergence, un ou plusieurs paramètres de saccade lors des tests de saccade, un ou plusieurs paramètres de vergence lors des tests combinés, un ou plusieurs paramètres de saccade lors des tests combinés, et/ou un ou plusieurs paramètres binoculaires lors du premier test du réflexe vestibulo-oculaire (RVO) de près et du deuxième test du RVO de loin, y compris une ou plusieurs parmi l'amplitude conjuguée ((œil gauche + œil droit)/2) des mouvements oculaires de crête à crête (de gauche à droite puis à gauche) et l'amplitude disconjuguée (œil gauche - œil droit) des mouvements oculaires de crête à crête (de gauche à droite puis à gauche).

8. Procédé selon l'une quelconque des revendications 1 à 7, dans lequel les paramètres posturaux calculés à l'étape c1) comprennent un ou plusieurs des paramètres de mouvement du corps suivants :
• Paramètres de balancement du corps pour les tests posturaux i) (test de vergence en position debout), ii) et iii) (tests de fixation de près et de loin) :
∘ Zone normalisée,
∘ Moyenne quadratique du balancement du corps médio-latéral,
∘ Moyenne quadratique du balancement du corps antéro-postérieur,
∘ Moyenne quadratique de la vitesse médio-latérale,
∘ Moyenne quadratique de la vitesse antéro-postérieure, et
∘ Fréquence de puissance moyenne ;
• Paramètres de rotation de la tête pour les tests posturaux iv) et v) (tests du RVO de près et de loin) :
∘ amplitude moyenne de rotation de la tête (gauche/droite ou haut/bas) et sa variabilité,
∘ amplitude moyenne du regard et sa variabilité, et
o gain moyen du RVO et sa variabilité.

9. Procédé selon l'une quelconque des revendications 1 à 6, dans lequel les paramètres de coordination binoculaire et les paramètres posturaux calculés à l'étape c1) comprennent :
• Des paramètres de vergence lors des deux tests de vergence, comprenant la latence de convergences et de divergences et l'amplitude totale de convergences et de divergences, comprenant en outre facultativement un ou plusieurs autres paramètres de vergence lors des deux tests de vergence ;
• Facultativement, des paramètres de saccade lors des deux tests de saccade, tels qu'un ou plusieurs paramètres de durée, un ou plusieurs paramètres d'amplitude et un ou plusieurs paramètres de vitesse ;
• Facultativement, des paramètres de vergence lors des deux tests combinés, tels qu'un ou plusieurs paramètres de durée, un ou plusieurs paramètres d'amplitude et un ou plusieurs paramètres de vitesse ;
• Facultativement, des paramètres de saccade lors des deux tests combinés, tels qu'un ou plusieurs paramètres de durée, un ou plusieurs paramètres d'amplitude et un ou plusieurs paramètres de vitesse ;
• Facultativement, un ou plusieurs paramètres binoculaires lors du premier test du réflexe vestibulo-oculaire (RVO) de près et du deuxième test du RVO de loin, y compris une ou plusieurs parmi l'amplitude conjuguée ((œil gauche + œil droit)/2) des mouvements oculaires de crête à crête (de gauche à droite puis à gauche) et l'amplitude disconjuguée (œil gauche - œil droit) des mouvements oculaires de crête à crête (de gauche à droite puis à gauche), et
• Des paramètres posturaux lors des tests posturaux, comprenant un ou plusieurs des éléments suivants :
o Paramètres de balancement du corps pour les tests posturaux i) (test de vergence en position debout), ii) et iii) (tests de fixation de près et de loin) :
▪ Zone normalisée,
▪ Moyenne quadratique du balancement du corps médio-latéral,
▪ Moyenne quadratique du balancement du corps antéro-postérieur,
▪ Moyenne quadratique de la vitesse médio-latérale,
▪ Moyenne quadratique du balancement de la vitesse antéro-postérieure, et
▪ Fréquence de puissance moyenne ;
∘ Paramètres de rotation de la tête pour les tests posturaux iv) et v) (tests du RVO de près et de loin) :
▪ amplitude moyenne de rotation de la tête (gauche/droite ou haut/bas) et sa variabilité,
▪ amplitude moyenne du regard et sa variabilité, et
▪ gain moyen du RVO et sa variabilité.

10. Procédé pour détecter des anomalies oculomotrices intrinsèques et des anomalies du contrôle postural chez un individu souffrant de vertige, comprenant :
a2) la fourniture d'enregistrements antérieurement obtenus des mouvements oculaires de l'œil gauche et de l'œil droit de l'individu lors de :
• tests oculomoteurs comprenant deux tests de vergence, et facultativement deux tests de saccade et/ou deux tests combinés, l'un étant réalisé avec l'individu en position assise et l'autre avec l'individu en position debout,
• tests posturaux comprenant :
iv. un premier test du réflexe vestibulo-oculaire (RVO) de près, consistant à tourner la tête à gauche et à droite tout en fixant une cible à une première distance comprise entre 20 et 50 cm, et
v. un deuxième test du réflexe vestibulo-oculaire (RVO) de loin, consistant à tourner la tête à gauche et à droite tout en fixant une cible à une deuxième distance comprise entre 100 et 200 cm,
b2) la fourniture d'enregistrements antérieurement obtenus du balancement du corps de l'individu lors de tests posturaux comprenant :
i) le test de vergence, et facultativement le test de saccade et/ou le test combiné, réalisé en position debout à l'étape a1),
ii) un premier test de fixation de près, consistant à fixer une cible pendant 20 à 40 secondes, de préférence 30 secondes, à une distance initiale comprise entre 20 et 50 cm,
iii) un deuxième test de fixation de loin, consistant à fixer une cible pendant 20 à 40 secondes, de préférence 30 secondes, à une deuxième distance comprise entre 100 et 200 cm,
iv) un premier test du réflexe vestibulo-oculaire (RVO) de près, consistant à tourner la tête à gauche et à droite tout en fixant une cible à une première distance comprise entre 20 et 50 cm, et
v) un deuxième test du réflexe vestibulo-oculaire (RVO) de loin, consistant à tourner la tête à gauche et à droite tout en fixant une cible à une deuxième distance comprise entre 100 et 200 cm,
c2) le calcul, à l'aide d'un dispositif de traitement numérique, de la ou des valeurs d'un ou plusieurs paramètres de coordination binoculaire à partir des enregistrements fournis à l'étape a2) et de la ou des valeurs d'un ou plusieurs paramètres posturaux à partir des enregistrements fournis à l'étape b2) ;
d2) la comparaison de la ou des valeurs calculées à l'étape c2) à une ou plusieurs valeurs de référence obtenues chez un ou plusieurs individus ne souffrant pas de vertige ; et
e2) la conclusion à la présence d'anomalies oculomotrices intrinsèques chez l'individu souffrant de vertige si un ou plusieurs paramètres de coordination binoculaire calculés à l'étape c2) sont significativement différents des une ou plusieurs valeurs de référence obtenues chez un ou plusieurs individus ne souffrant pas de vertige ; et
f2) la conclusion à la présence d'anomalies du contrôle postural chez l'individu souffrant de vertige si un ou plusieurs paramètres posturaux calculés à l'étape c2) sont significativement différents des une ou plusieurs valeurs de référence obtenues chez un ou plusieurs individus ne souffrant pas de vertige ;
dans lequel les deux tests de vergence à partir desquels les enregistrements sont fournis à l'étape a2) ont été effectués à l'aide d'un dispositif de stimulation motrice binoculaire configuré pour stimuler spécifiquement et physiquement les vergences et les saccades, comprenant des cibles visuelles ou audiovisuelles placées dans l'espace réel à des excentricités et des profondeurs d'une surface ; dans lequel chacun des deux tests de vergence dont les enregistrements sont fournis comprenait 30 à 50 essais de :
• stimulation visuelle et/ou audiovisuelle de l'individu à un point situé au niveau des yeux, dans l'axe central entre l'œil gauche et l'œil droit, à une première distance, et
• stimulation visuelle et/ou audiovisuelle de l'individu à un autre point situé au niveau des yeux, dans l'axe central entre l'œil gauche et l'œil droit, à une distance différente nécessitant un mouvement de convergence ou un mouvement de divergence, la moitié des essais nécessitant un mouvement de convergence, l'autre moitié un mouvement de divergence, les essais nécessitant des mouvements de convergence et de divergence étant entrelacés.

11. Procédé selon la revendication 10, dans lequel les enregistrements fournis à l'étape a2) ont été obtenus comme à l'étape a1) ou b1) du procédé selon l'une quelconque des revendications 1 à 9 et les enregistrements fournis à l'étape b2) ont été obtenus comme à l'étape b1) du procédé selon l'une quelconque des revendications 1 à 9.

12. Procédé selon l'une quelconque des revendications 1 à 11, dans lequel l'individu souffrant de vertige souffre d'un vertige d'origine organique.

13. Procédé selon l'une quelconque des revendications 1 à 11, dans lequel l'individu souffrant de vertige souffre d'un vertige d'origine fonctionnelle.

14. Procédé selon la revendication 13, dans lequel l'individu souffrant de vertige souffre d'asthénopie vestibulaire ou de la maladie de Ménière.
